(19) Europäisches Patentamt
European Patent Office
Office européen des brevets

(11) Numéro de publication: **0 531 212 B1**

# (12) FASCICULE DE BREVET EUROPEEN

(45) Date de publication du fascicule du brevet: **13.12.95**

(51) Int. Cl.[6]: **C07J 5/00**, C07J 33/00, C07J 13/00, C07J 21/00

(21) Numéro de dépôt: **92402406.0**

(22) Date de dépôt: **03.09.92**

## (54) Nouveau procédé de préparation de l'hydrocortisone et nouveaux intermédiaires

(30) Priorité: **06.09.91 FR 9111052**

(43) Date de publication de la demande:
**10.03.93 Bulletin 93/10**

(45) Mention de la délivrance du brevet:
**13.12.95 Bulletin 95/50**

(84) Etats contractants désignés:
**AT BE CH DE DK ES FR GB GR IE IT LI LU NL PT SE**

(56) Documents cités:
**EP-A- 0 336 521**
**FR-A- 2 318 647**
**US-A- 2 744 110**

(73) Titulaire: **ROUSSEL UCLAF**
**102 Route de Noisy**
**F-93230 Romainville (FR)**

(72) Inventeur: **Brion, Francis**
**10 bis, rue d'Avron**
**F-93220 Gagny (FR)**
Inventeur: **Buendia, Jean**
**3 bis, Impasse Emilie**
**F-94170 Le Perreux sur Marne (FR)**
Inventeur: **Diolez, Christian**
**6, Allée des Rouges-Gorges**
**F-91000 Palaiseau (FR)**
Inventeur: **Vivat, Michel**
**14, Chemin de l'Autostrade**
**F-77400 Lagny sur Marne (FR)**

(74) Mandataire: **Vieillefosse, Jean-Claude et al**
**ROUSSEL UCLAF,**
**Département des Brevets,**
**111, Route de Noisy**
**F-93235 Romainville Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention a pour objet un nouveau procédé de préparation de l'hydrocortisone et des nouveaux intermédiaires.

L'invention a ainsi pour objet un procédé de préparation de l'hydrocortisone de formule :

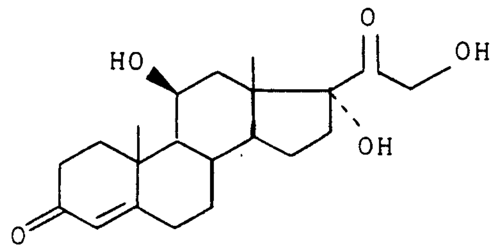

(I)

caractérisé en ce que ou bien l'on soumet une halohydrine de formule (II) :

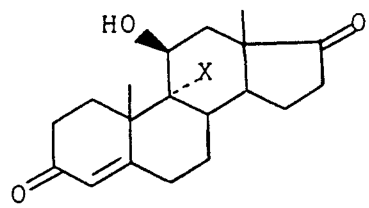

(II)

dans laquelle X représente un atome de chlore, de brome ou d'iode, à une réaction de réarrangement en présence d'un alcool ou d'un polyalcool, pour obtenir après traitement par un acide, le produit de formule (III) :

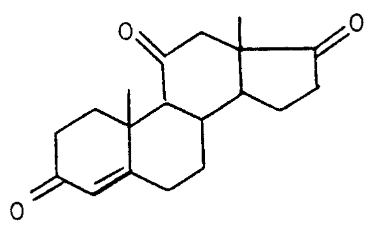

(III)

dont on protège sélectivement la fonction 3-oxo par action d'un thiol ou d'un dithiol de formule :

$HO-(CH_2)_n-SH$ ou $HS-(CH_2)_n-SH$

dans laquelle n est égal à 2 ou 3, pour obtenir un composé de formule (IV) :

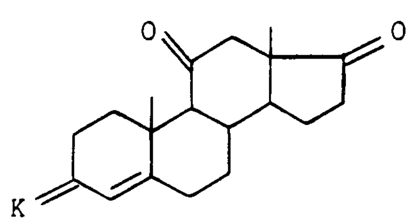

(IV)

dans laquelle K représente un groupement protecteur du radical 3-oxo de formule

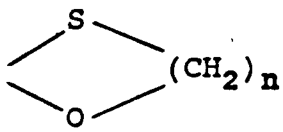

ou

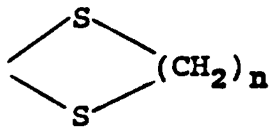

dans laquelle n est défini comme précédemment, ou bien l'on traite un composé de formule (II) telle que définie précédemment par un agent de blocage sélectif de la fonction 3-oxo tel que défini précédemment, pour obtenir un composé de formule (V) :

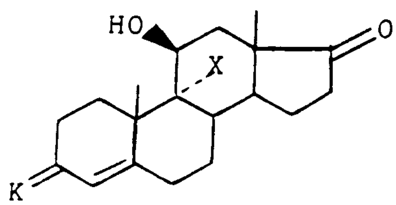

**(V)**

dans laquelle X et K sont définis comme précédemment, que l'on soumet à une réaction de réarrangement en présence d'un alcool ou d'un polyalcool, pour obtenir après traitement par un acide, un composé de formule (IV) telle que défini précédemment, puis traite ledit composé de formule (IV) par un trihaloacétate de formule

$Hal_3C\text{-}CO_2R$

dans laquelle Hal représente un atome de chlore ou de brome et R représente un radical alkyle renfermant de 1 à 6 atomes de carbone, un radical aralkyle renfermant de 7 à 15 atomes de carbone ou un reste silylé, en présence de zinc et d'un acide de Lewis, pour obtenir un composé de formule (VI) :

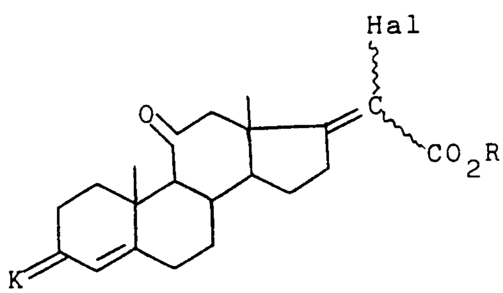

**(VI)**

dans laquelle K, Hal et R sont définis comme précédemment, que l'on traite, en milieu basique, par un phénol de formule :

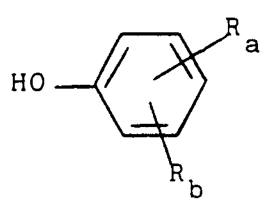

dans laquelle $R_a$ et $R_b$, identiques ou différents, représentent un atome d'hydrogène, un radical hydroxy, un radical alkyle renfermant de 1 à 4 atomes de carbone ou un radical alkoxy renfermant de 1 à 4 atomes de carbone, pour obtenir un composé de formule (VII) :

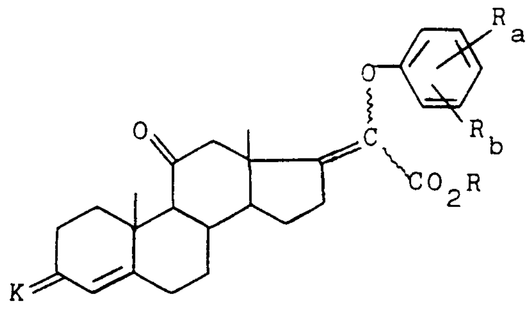

(VII)

dans laquelle K, R, $R_a$ et $R_b$ sont définis comme précédemment, que l'on soumet à l'action d'un agent réducteur, pour obtenir un composé de formule (VIII) :

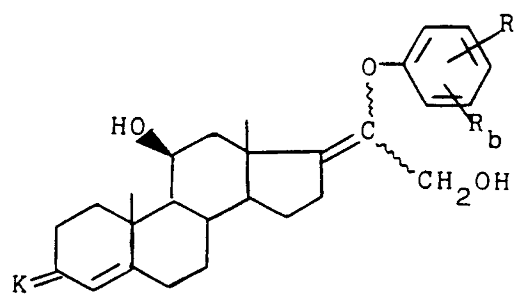

(VIII)

dans laquelle K, $R_a$ et $R_b$ sont définis comme précédemment, dont on déprotège la fonction 3-oxo pour obtenir un composé de formule (IX) :

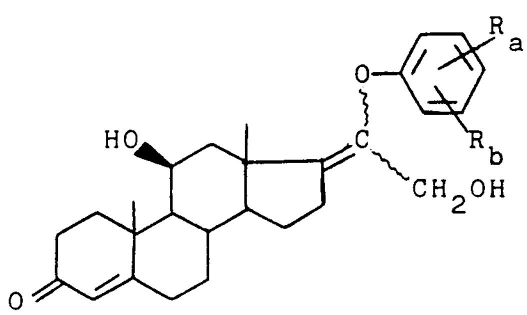

(IX)

dans laquelle $R_a$ et $R_b$ sont définis comme précédemment, que l'on traite par un agent d'époxydation, pour obtenir un composé de formule (X) :

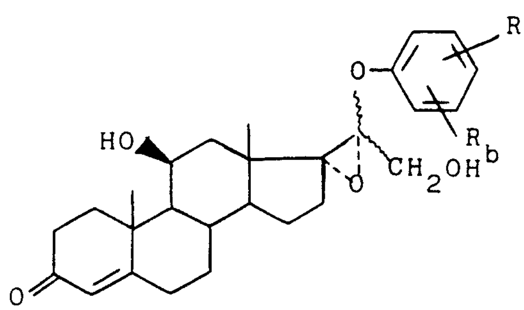

(X)

dans laquelle $R_a$ et $R_b$ sont définis comme précédemment, que l'on hydrolyse en milieu acide, pour obtenir le composé de formule (I) attendu.

L'invention a donc notamment pour objet un procédé de préparation de l'hydrocortisone tel que défini précédemment, caractérisé en ce que l'on soumet une halohydrine de formule (II) :

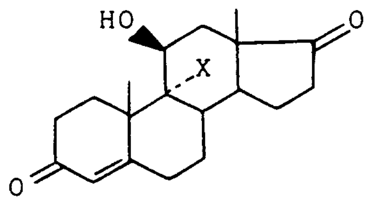

(II)

dans laquelle X est défini comme précédemment, à une réaction de réarrangement en présence d'un alcool ou d'un polyalcool pour obtenir après traitement par un acide, le composé de formule (III) :

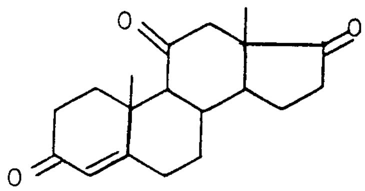

(III)

dont on protège sélectivement la fonction 3-oxo par action d'un thiol ou d'un dithiol de formule :

$HO-(CH_2)_n-SH$ ou $HS-(CH_2)_n-SH$

dans laquelle n est défini comme ci-dessus, pour obtenir un composé de formule (IV) :

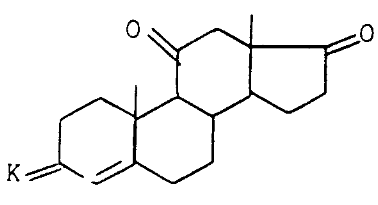

(IV)

dans laquelle K est défini comme ci-dessus, que l'on traite par un réactif de formule $Hal_3C-CO_2R$ dans laquelle Hal et R sont définis comme précédemment puis poursuit la synthèse comme décrit précédemment, ainsi qu'un procédé caractérisé en ce que l'on traite un composé de formule (II) telle que définie précédemment, par un agent de protection sélectif de la fonction 3-oxo tel que défini précédemment, pour obtenir un composé de formule (V) :

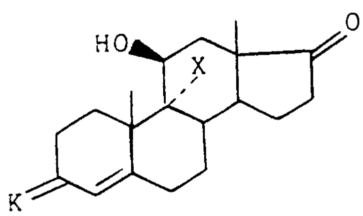

(V)

dans laquelle X et K sont définis comme précédemment, que l'on soumet à une réaction de réarrangement en présence d'un alcool ou d'un polyalcool pour obtenir après traitement par un acide, un composé de formule (IV) telle que définie précédemment, puis traite ledit composé de formule (IV) par un réactif de formule

$Hal_3C\text{-}CO_2R$

puis poursuit la synthèse comme décrit précédemment.

Lorsque R représente un radical alkyle, il s'agit de préférence d'un radical méthyle, éthyle, propyle, isopropyle, butyle, sec-butyle, tert-butyle, pentyle ou hexyle.

Lorsque R représente un radical aralkyle, il s'agit de préférence d'un radical benzyle ou phénéthyle.

Lorsque R représente un reste silyle, il s'agit par exemple d'un reste trialkylsilyle tel que triméthylsilyle, tert-butyl diméthylsilyle, ou encore, par exemple, d'un reste triphénylsilyle ou diphényl tert-butylsilyle.

Lorsque $R_a$ et $R_b$ représentent un radical alkyle, il s'agit d'un radical éthyle, propyle linéaire ou ramifié, butyle linéaire ou ramifié ou, de préférence, méthyle.

Lorsque $R_a$ et $R_b$ représentent un radical alkoxy, il s'agit d'un radical éthoxy, propoxy linéaire ou ramifié, butoxy linéaire ou ramifié ou, de préférence, méthoxy.

L'invention a plus particulièrement pour objet un procédé tel que défini précédemment, caractérisé en ce que dans le composé de formule (II), X représente un atome de brome.

La réaction de réarrangement de l'halohydrine est de préférence effectuée en présence du glycérol ou un diol tel que le propylène glycol ou, de préférence l'éthylène glycol, utilisé en excès, par chauffage à une température inférieure à 100 ° C. Il peut être avantageux d'opérer en présence d'un cosolvant, de préférence de point d'ébullition inférieur à 100 ° C, au reflux de celui-ci. Le cosolvant est un solvant inerte dans les conditions de la réaction, par exemple l'acétate d'éthyle.

Le traitement acide est réalisé par un acide aqueux, par exemple l'acide chlorhydrique, bromhydrique ou sulfurique.

La protection de la fonction 3-oxo est réalisée par action d'un dithiol en milieu acide, notamment de l'éthane dithiol en présence d'acide chlorhydrique ou bromhydrique concentré, en quantité catalytique, ou encore en présence d'un acide de Lewis tel que le chlorure de zinc, le tétrachlorure de titane ou le trifluorure de bore, de préférence sous forme d'étherate.

L'invention a notamment pour objet un procédé tel que défini précédemment, dans lequel on effectue le blocage de la fonction 3-oxo, puis le réarrangement de l'halohydrine, caractérisé en ce que l'on opère selon une méthode dite "one pot", c'est à dire sans isolement de l'intermédiaire de formule (V).

Le réarrangement de l'halohydrine est facilité par le blocage intermédiaire des fonctions oxo en 3 ou en 3 et 17, ce qui autorise l'emploi de conditions opératoires très douces.

On peut signaler à titre indicatif, que la conséquence du blocage paraît être la labilisation de la liaison carbonehalogène en 9 qui facilite donc le réarrangement.

L'acide de Lewis utilisé dans la réaction du composé de formule (IV) avec le trihaloacétate est, par exemple, le chlorure de zinc, le chlorure d'aluminium, le chlorure de diéthylaluminium, ou, de préférence le tétrachlorure de titane.

L'invention a notamment pour objet un procédé tel que défini précédemment, caractérisé en ce que l'on utilise un trihaloacétate d'alkyle et tout particulièrement un trichloroacétate de méthyle ou d'éthyle.

On opère de préférence au sein d'un éther cyclique tel que le tétrahydrofuranne ou le dioxanne.

L'action du phénol sur le composé de formule (VI) est réalisée en présence d'une base qui peut être par exemple un hydroxyde ou un carbonate alcalin ou alcalino terreux, notamment de sodium, potassium, baryum ou calcium, un hydrure, un alcoolate ou un amidure alcalin, notamment de sodium, potassium ou lithium ou encore un alkyl lithium, notamment le butyl lithium.

On opère au sein d'un solvant organique, par exemple une cétone telle que l'acétone ou la méthyléthyl cétone, le cas échéant en mélange avec un solvant halogéné tel que le chlorure de méthylène ou avec un éther tel que le dioxanne ou le tétrahydrofuranne.

L'invention a notamment pour objet un procédé tel que défini précédemment, caractérisé en ce que dans le phénol de formule

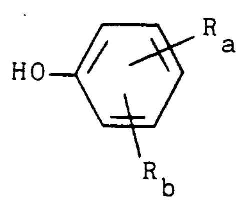

,

$R_a$ et $R_b$ représentent un atome d'hydrogène, un radical hydroxy ou un radical méthyle.

L'agent réducteur peut être notamment un hydrure, de préférence d'aluminium, par exemple l'hydrure double de lithium et d'aluminium, l'hydrure de diéthyl sodium aluminium, l'hydrure de diisobutyl aluminium ou encore le dihydro bis(2-méthoxyéthoxy) aluminate de sodium. On opère par exemple dans le toluène ou le tétrahydrofuranne.

L'agent réducteur peut encore notamment être un borohydrure alcalin, par exemple le borohydrure de sodium, catalysé le cas échéant, par un sel de lithium, ou le borohydrure de lithium.

La déprotection de la fonction 3-oxo est réalisée par action de l'iode en présence d'une base par exemple un bicarbonate alcalin, ou par action de l'iode en quantité catalytique, en présence d'un agent oxydant, notamment l'eau oxygénée, par action de l'iodure de méthyle, de l'acide glyoxylique, ou encore de sels de métaux, tels que le mercure ou le cadmium. On peut, en général, opérer dans un solvant tel qu'un alcanol inférieur, par exemple le méthanol ou l'éthanol, en mélange avec un solvant halogéné, par exemple le chlorure de méthylène, en présence d'eau.

L'agent d'époxydation peut être un peracide tel que l'acide métachloroperbenzoïque, l'acide perphtalique, l'acide pertungstique ou encore l'eau oxygénée utilisée seule ou en présence d'hexachloro ou d'hexafluoroacétone.

L'agent d'époxydation peut encore être un hydroperoxyde tel que l'hydroperoxyde de tertbutyle, utilisé en présence d'acétyl acétonate de vanadium ou autres métaux tel que le molybdène, en quantité catalytique.

On peut opérer au sein d'un solvant organique tel que le chlorure de méthylène, le tétrachlorure de carbone, le chloroforme, le méthanol, le tétrahydrofuranne, le dioxanne, le toluène ou l'acétate d'éthyle, le cas échéant en présence d'eau. On peut également opérer en milieu tamponné, par exemple par du phosphate disodique ou le mélange phosphate trisodique-acide phosphorique.

L'hydrolyse de l'époxyde en 17,20 est effectuée par action d'un acide aqueux, l'acide étant notamment un acide minéral tel que l'acide chlorhydrique, l'acide sulfurique ou l'acide nitrique. On peut également opérer en milieu tamponné, tel que ceux mentionnés ci-dessus.

La nouvelle synthèse de l'hydrocortisone selon l'invention présente un certain nombre d'avantages, résumés dans les points suivants :

- Le réarrangement conduisant du composé 11-OH au composé 11-céto via l'halohydrine intermédiaire, est effectué dans des conditions beaucoup plus douces que celles décrites dans le brevet européen 30368, ce qui représente à la fois un avantage au niveau du rendement de la réaction, car la formation de produits secondaires ou de dégradation est limitée, et un avantage au niveau industriel, dans la mesure où la synthèse est d'autant plus économique.
- Le blocage en position 3 est remarquablement sélectif, au contraire des blocages connus par des éthers d'énols et des cétals qui conduisent à des mélanges de produits bloqués en 3 et en 3,17.
- Le blocage en position 3 selon l'invention est en outre très stable dans les conditions réactionnelles utilisées, qu'elles soient acides ou basiques, et son élimination en cours de synthèse, notamment par action de l'iode en milieu basique ou de l'iode en quantité catalytique, en milieu oxydant doux, est très aisée.
- L'accès à l'hydrocortisone est possible sans passer par un stade d'hydroxylation en position 11, ce qui est le cas notamment dans les synthèses connues utilisant au départ l'androstène dione. Ceci améliore d'autant le rendement global du procédé.

L'invention a enfin pour objet, à titre de composés industriels nouveaux et notamment à titre d'intermédiaires nécessaires à la mise en oeuvre du procédé ci-dessus,

- les composés de formule (F) :

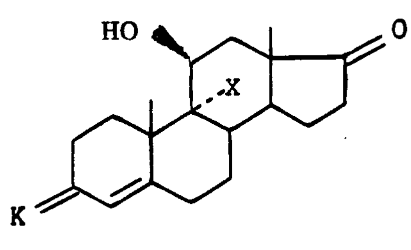

(F)

dans laquelle K représente un groupement protecteur du radical oxo de formule

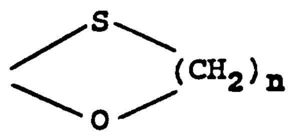

ou de préférence

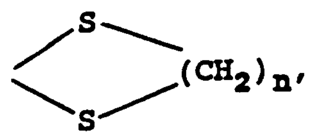

dans laquelle n est égal à 2 ou 3 et notamment égal à 2et X représente un atome de chlore, de brome ou d'iode et notamment un atome de brome.

- les composés de formule (G) :

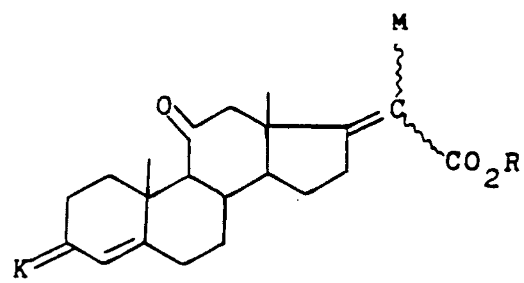

(G)

dans laquelle K représente un groupement protecteur du radical oxo de formule

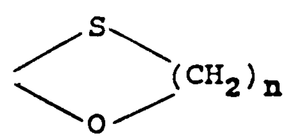

ou de préférence

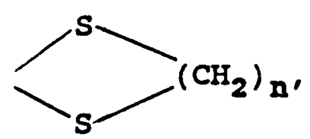

dans laquelle n est égal à 2 ou 3 et notamment égal à 2 et M représente soit un atome de chlore ou de brome et notamment un atome de chlore soit un groupement

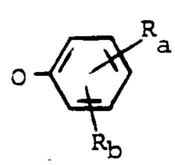

dans lequel $R_a$ et $R_b$ ont la signification indiquée ci dessus et représentent notamment un atome d'hydrogène, un radical hydroxy ou un radical méthyle et R a la signification indiquée ci-dessus et notamment méthyle ou éthyle, étant etendu que, lorsque R répresente un reste silyle, il s'agit d'un reste trialkylsilyle, ou d'un reste triphénylsilyle ou diphényle t-butylsilyle, ainsi que:

- les composés de formule (J) :

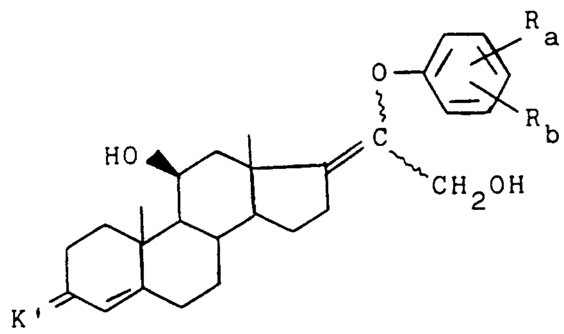

(J)

dans laquelle soit K' représente un atome d'oxygène ou un radical K, lequel représente un groupement protecteur du radical oxo de formule

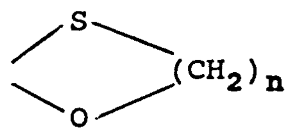

ou de préférence

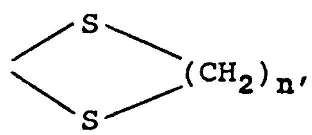

dans laquelle n est égal à 2 ou 3 et notamment égal à 2, le trait pointillé en position 17 représente une liaison, $R_a$ et $R_b$ ont la signification ci-dessus et représentent notamment un atome d'hydrogène, un radical hydroxy ou un radical méthyle, soit K' représente un atome d'oxygène, le trait pointillé en position 17 représente une fonction époxy, $R_a$ et $R_b$ ont la signification indiquée ci-dessus.

Les composés de formule (II) sont décrits notamment dans le brevet US 3.072.684, le document EP-A-336, 521 décrit un procédé de préparation des dérivés corticoïdes, et EP-A-30, 368 décrit le réarrangement des halohydrines sous conditions plus dures.

L'exemple suivant illustre l'invention sans toutefois la limiter.

**Exemple : Hydrocortisone**

Stade A : 3-[(1,2-éthanediyl) mercaptole] cyclique d'Androst-4-ène 3,11,17-trione.

**a) Androst-4-ène 3,11,17-trione.**

On mélange sous gaz inerte à température ambiante 1,05 g de 9alpha-bromo 11béta-hydroxy androst-4-ène 3,17-dione, 7,5 $cm^3$ d'acétate d'éthyle et 2,5 $cm^3$ d'éthylène glycol. On porte au reflux sous agitation pendant 10 heures, refroidit puis ajoute 10 $cm^3$ d'acide chlorhydrique 2N et 10 $cm^3$ d'eau, agite pendant 20 heures puis élimine l'acétate d'éthyle sous pression réduite. Après un relargage par du chlorure de sodium on refroidit vers 0 ° C, essore les cristaux, les lave à l'eau et les sèche. On les reprend au chlorure de méthylène, ajoute de l'acétate d'éthyle, évapore le chlorure de méthylène, glace la solution et essore les cristaux puis les sèche. On obtient 0,52 g de produit attendu F = 221 ° C. Par extraction de la phase aqueuse au chlorure de méthylène et chromatographie sur silice du produit brut, en éluant au mélange chlorure de méthylèneacétate d'éthyle (95-5), on obtient, après cristallisation dans l'acétate d'éthyle, 0,097 g de produit attendu F = 220 ° C.

**b) 3-[(1,2-éthanediyl) mercaptole] cyclique d'androst-4-ène 3,11,17-trione.**

On mélange sous gaz inerte à température ambiante 100 $cm^3$ de méthanol, 5 g de produit obtenu comme décrit ci-dessus, 1,8 $cm^3$ d'éthane dithiol et 2,5 $cm^3$ d'éthérate de trifluorure de bore. Après 1 h 30 sous agitation on évapore le méthanol, reprend au chlorure de méthylène, lave par une solution aqueuse saturée de bicarbonate de sodium, à l'eau et sèche puis amène à sec. On cristallise le produit obtenu dans l'hexane et obtient 5,99 g de produit attendu F = 160 ° C.
Spectre RMN ($CDCl_3$ 90 MHz ppm)
18-$CH_3$ : 0,85 ; 19-$CH_3$ : 1,27 ; thiocétal : 3,17 à 3,47 ; $H_4$ : 5,6
Spectre IR ($CHCl_3$)
Absence de delta4 3-one ; absorptions à 1645 $cm^{-1}$ (C = C), 1709 $cm^{-1}$ (C = O en 11), 1740 $cm^{-1}$ (C = O en 17).

Stade A' : 3-[(1,2-éthanediyl) mercaptole] cyclique d'Androst-4-ène 3,11,17-trione.

On mélange sous gaz inerte 4 g de 9alpha-bromo 11béta-hydroxy androst-4-ène 3,20-dione et 40 $cm^3$ d'acétate d'éthyle puis ajoute à température ambiante 0,9 $cm^3$ d'éthane dithiol. On ajoute ensuite lentement 0,09 $cm^3$ d'acide chlorhydrique à 22 ° Bé puis maintient sous agitation pendant 6 heures. On introduit ensuite 9,3 $cm^3$ d'éthylène glycol, porte au reflux pendant 20 heures puis refroidit à 20 ° C et verse le mélange réactionnel dans un mélange de 40 $cm^3$ d'acide chlorhydrique 2N et 40 $cm^3$ d'eau. On agite pendant 16 heures puis élimine l'acétate d'éthyle sous pression réduite (20 mm/Hg) à 35 ° C maximum. On refroidit ensuite la suspension à 0, + 5 ° C, maintient sous agitation pendant 1 heure et essore les cristaux. On les lave à l'eau puis on les sèche et les chromatographie sur silice en éluant au mélange hexane-dioxane (9-1). On obtient 3,2 g de produit attendu.
Spectre RMN ($CDCl_3$ 300 MHz ppm)
18-$CH_3$ : 0,84(s) ; 19-$CH_3$ : 1,26(s) ; thiocétal : 3,15 à 3,4 $H_4$ : 5,57 ; squelette : 1,1 à 2,61(m)
Spectre IR ($CHCl_3$)
Absorptions à 1741-1709 $cm^{-1}$ (cétones) ; 1641 $cm^{-1}$ (C = C)

Stade B : 20-chloro-3,3-[1,2-éthanediyl bis(thio)]-11-oxo-pregna-4,17(20)-dien-21-oate de méthyle.

On mélange sous atmosphère inerte 100 $cm^3$ de tétrahydrofuranne et 12,55 g de poudre de zinc. On ajoute lentement, à -10 °/-15 ° C, 7,9 $cm^3$ de tétrachlorure de titane, puis un mélange de 100 $cm^3$ de tétrahydrofuranne, 8,6 $cm^3$ de trichloracétate de méthyle et 18 g de produit obtenu comme décrit au stade A ou A'. On laisse remonter la température puis maintient sous agitation à température ambiante pendant 1 h 30. On ajoute ensuite à + 10/ + 15 ° C, 100 $cm^3$ de mélange eau-pyridine (4-1), agite le mélange pendant 1 heure en laissant remonter la température puis ajoute 100 $cm^3$ de mélange eau-acide chlorhydrique concentré (6-4). On agite pendant 15 minutes, extrait au chlorure de méthylène, lave la phase organique à l'eau, la sèche et évapore le solvant. On dissout les cristaux dans du chlorure de méthylène, ajoute de l'éther isopropylique, évapore le chlorure de méthylène, refroidit et essore les cristaux. On chromatographie les liqueurs mères sur silice en éluant au mélange cyclohexane-acétate d'éthyle (8-2). On récupère au total 21,8 g de produit attendu F≈175 ° C.
Spectre IR ($CHCl_3$)
Absorptions à 1715 $cm^{-1}$ et 1730 $cm^{-1}$ (C = O) max. 1705 $cm^{-1}$ 1643 $cm^{-1}$ (C = C delta4) et 1610 $cm^{-1}$ (C = C)
Spectre RMN ($CDCl_3$ 90 MHz ppm)
Mélange d'isomères 20 Cl
18-$CH_3$ : 1,02-0,98 ; 19-$CH_3$ : 1,25 ; thiocétal : 3,33 ; $CH_3$-ester : 3,83-3,82 ; $H_4$ : 5,58.

Stade C : 20-phénoxy-3,3-[1,2-éthanediyl bis(thio)] 11-oxo pregna-4,17(20)-dièn-21-oate de méthyle.

On porte au reflux sous atmosphère inerte le mélange de 18 g de phénol, 150 $cm^3$ de butanone, 30 g de produit obtenu selon le procédé décrit au stade B et 17,7 g de carbonate de potassium. Après 16 heures on verse le mélange dans un mélange de 100 $cm^3$ d'eau, 90 g de glace et 10 $cm^3$ de soude 10N. On extrait au chlorure de méthylène, lave la phase organique à l'eau et la concentre. On reprend par du méthanol, laisse refroidir lentement et essore les cristaux puis les sèche. On obtient en deux jets 27,4 g de produit attendu F≈208-210 ° C.
Spectre IR ($CHCl_3$)

Absorption à 1592-1491 $cm^{-1}$ (Aromatique type $C_6H_5$-O-) ; 1714-1705 $cm^{-1}$ (C=O) ; 1646 $cm^{-1}$ (C=C)
Spectre RMN ($CDCl_3$ 90 MHz ppm)
18-$CH_3$ : 0,9 ; 19-$CH_3$ : 1,21 ; thiocétal : 3,33 ; $CH_3$ ester : 3,63 ; $H_4$ : 5,58 ; $C_6H_5$ : 6,81 à 7,39
Mélange d'isomères 20-0-$C_6H_5$

Stade D : (11béta) 3,3-[(1,2-éthanediyl) mercaptole] cyclique de 20-phénoxy 11,21-dihydroxy-pregna-4,17-(20)-dièn-3-one.

On mélange sous gaz inerte 20 g de produit obtenu au stade C et 200 $cm^3$ de toluène, refroidit à -25°C et introduit lentement 110 $cm^3$ d'une solution à 20 % de diisobutyl aluminium hydrure dans le toluène. On laisse remonter la température à + 10°C, agite pendant 1 heure puis refroidit à nouveau à -15°C. On ajoute lentement 10 $cm^3$ de méthanol, laisse remonter jusqu'à 0°C et ajoute lentement 200 $cm^3$ d'acide chlorhydrique 2N. On décante, lave la phase organique à l'eau, la sèche et évapore le solvant. On chromatographie le résidu sur silice en éluant au mélange toluène-acétate d'éthyle (9-1). On obtient 17,4 g de produit attendu.
Spectre IR ($CHCl_3$)
Absorption à 1490-1596 $cm^{-1}$ ($C_6H_5$-0-C) ; 1644 $cm^{-1}$ (C=C delta4) et 1682 $cm^{-1}$ (C=C) ; 3612 $cm^{-1}$ (OH libre)
Spectre RMN ($CDCl_3$-$C_5D_5N$) (90 MHz ppm)
18-$CH_3$ : 1,17 ; 19-$CH_3$ : 1,29 ; thiocétal : 3,33 ; $CH_2OH$ : 4,15 ; $H_{11}$ : 4,32 ; $H_4$ : 5,45

Stade E : (11béta) 11,21-dihydroxy 20-phénoxy pregna-4,17 (20) dièn-3-one.

On dissout 5 g du produit obtenu ci-dessus dans 10 $cm^3$ de chlorure de méthylène et 30 $cm^3$ de méthanol, on ajoute 2,5 $cm^3$ d'eau déminéralisée puis 0,3 g d'iode. On obtient un pH de 1,5 et on introduit en 15 minutes 1,4 $cm^3$ d'eau oxygénée à 50 %.

On neutralise le pouvoir oxydant par ajout de 2 g de thiosulfate de sodium. On ajoute ensuite 5 g de clarcel, filtre et concentre à sec sous pression réduite. On dissout l'extrait sec dans le chlorure de méthylène, lave avec une solution de 1 g de thiosulfate de sodium dans 25 $cm^3$ d'eau, décante, sèche et concentre à sec sous pression réduite et recueille 4,8 g de produit brut attendu.

On purifie 1,8 g de ce produit par chromatographie sur silice (éluant chlorure de méthylène-isopropanol (97,5-2,5)) et obtient 1,7 g de produit recherché. F=188°C.
Spectre IR ($CHCl_3$)
Absorptions à 3613 $cm^{-1}$ (OH) ; 1662, 1617 et 868 $cm^{-1}$ (delta4-3oxo) ; 1597-1491 $cm^{-1}$ (-O-$C_6H_5$)
Spectre RMN ($CDCl_3$ - $C_5D_5N$ - 90 MHz ppm)
18-$CH_3$ : 1,17 ; 19-$CH_3$ : 1,42 ; $H_{11}$ : 4,27 ; $H_4$ : 5,67 ; $CH_2OH$ : 4,11 ; $C_6H_5$ : 6,87 à 7,37

Stade F : (11béta) 11,21-dihydroxy 17,20-époxy 20-phénoxy pregna-4-en-3-one.

On mélange sous gaz inerte 1,0 g de produit obtenu au stade E, 10 $cm^3$ d'acétate d'éthyle et 5 $cm^3$ d'eau. On y ajoute 0,5 g de phosphate disodique, 0,65 g d'acide perphtalique et 0,75 $cm^3$ d'eau oxygénée à 50 %, agite pendant 3 h 15 minutes, rajoute 0,15 g de phosphate disodique et 0,20 g d'acide perphtalique et agite pendant 1 h 15 minutes. On ajoute ensuite 20 $cm^3$ d'acétate d'éthyle et 9 $cm^3$ de soude 1N, agite 5 minutes, décante, lave la phase organique à l'eau et à l'eau additionnée de bisulfite de sodium et d'acide sulfurique 1N, sèche et évapore à sec. On obtient 1,05 g de produit attendu brut et instable, utilisé tel quel pour le stade suivant.
rf : 0,28 (silice - $CH_2Cl_2$/Dioxanne - 90/10)
Spectre IR ($CHCl_3$)
Absorption à 3613 $cm^{-1}$ (OH) ; 1662 et 1616 $cm^{-1}$ (delta4-3-one); 1600, 1590 et 1494 $cm^{-1}$ (aromatique)
Spectre RMN ($CDCl_3$ 300 MHz ppm)
18-$CH_3$ : 1,29(s) ; 19-$CH_3$ : 1,43(s) ; -C-$CH_2$-O- : 3,49 (dd) et 4,20 (dd) ; $H_{11}$ éq. : 4,32 ; $H_4$ : 5,68 ; H de O-$C_6H_5$ : para 7,06(t), ortho 7,13(d) et méta 7,29(t)

Stade G : Hydrocortisone.

On mélange sous gaz inerte 5 $cm^3$ de méthanol, 3 $cm^3$ d'eau et 0,08 $cm^3$ d'acide sulfurique 1N (pH 2). On ajoute à température ambiante 0,525 g de produit obtenu au stade F et maintient sous agitation pendant 16 heures. On neutralise par addition de bicarbonate de sodium et extrait au chlorure de méthylène. On

sèche la phase,organique et l'évapore à sec. On reprend le résidu par du chlorure de méthylène à 5 % de méthanol à chaud puis concentre jusqu'à début de cristallisation. On laisse refroidir, essore les cristaux et les sèche. On concentre les liqueurs mères et chromatographie le résidu sur silice en éluant au mélange chlorure de méthylèneméthanol (95-5). On obtient au total 0,318 g d'hydrocortisone attendue F = 224 ° C. Alpha$_D^{20}$ = + 164 ° ± 2 ° 5 (C = 1 % éthanol).
Spectre IR (nujol)
Absorption à 3430 $cm^{-1}$ (OH), 1710 $cm^{-1}$ (C = O), 1642, 1630 et 1610 $cm^{-1}$ (delta4 3-one).

**Revendications**

**Revendications pour les Etats contractants suivants : AT, BE, CH, LI, DE, DK, FR, GB, GR, IT, LU, NL, PT, SE, IE**

**1.** Procédé de préparation de l'hydrocortisone de formule :

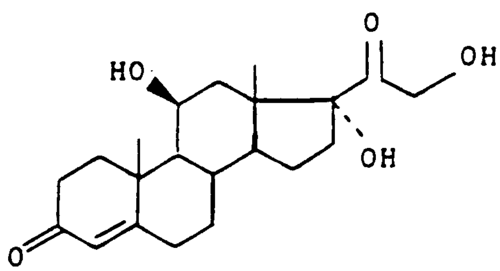

(I)

caractérisé en ce que ou bien l'on soumet une halohydrine de formule (II) :

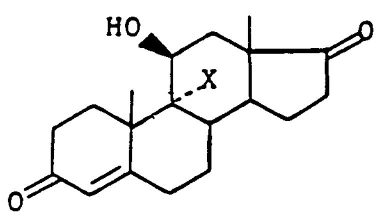

(II)

dans laquelle X représente un atome de chlore, de brome ou d'iode, à une réaction de réarrangement en présence d'un alcool ou d'un polyalcool, pour obtenir après traitement par un acide, le produit de formule (III) :

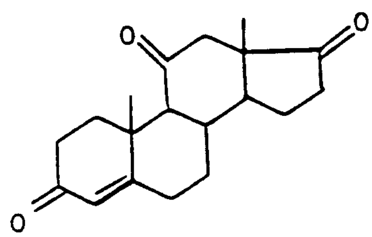

(III)

dont on protège sélectivement la fonction 3-oxo par action d'un thiol ou d'un dithiol de formule :

$HO-(CH_2)_n-SH$ ou $HS-(CH_2)_n-SH$

dans laquelle n est égal à 2 ou 3, pour obtenir un composé de formule (IV) :

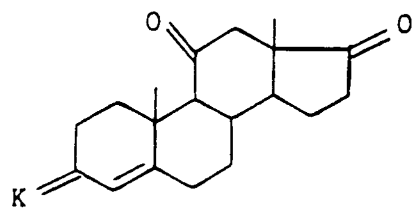

(IV)

dans laquelle K représente un groupement protecteur du radical 3-oxo de formule

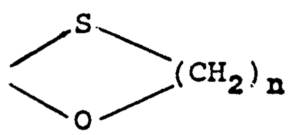 ou 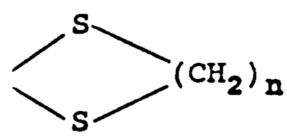

dans laquelle n est défini comme précédemment, ou bien l'on traite un composé de formule (II) telle que définie précédemment par un agent de blocage sélectif de la fonction 3-oxo tel que défini précédemment, pour obtenir un composé de formule (V) :

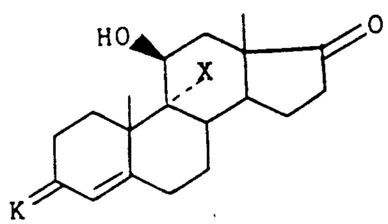

(V)

dans laquelle X et K sont définis comme précédemment, que l'on soumet à une réaction de réarrangement en présence d'un alcool ou d'un polyalcool, pour obtenir après traitement par un acide, un composé de formule (IV) telle que défini précédemment, puis traite ledit composé de formule (IV) par un trihaloacétate de formule

$Hal_3C\text{-}CO_2R$

dans laquelle Hal représente un atome de chlore ou de brome et R représente un radical alkyle renfermant de 1 à 6 atomes de carbone, un radical aralkyle renfermant de 7 à 15 atomes de carbone ou un reste silylé, en présence de zinc et d'un acide de Lewis, pour obtenir un composé de formule (VI) :

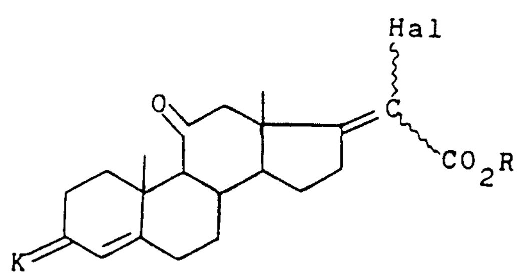

(VI)

dans laquelle K, Hal et R sont définis comme précédemment, que l'on traite, en milieu basique, par un phénol de formule :

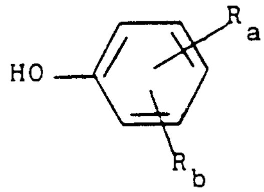

dans laquelle $R_a$ et $R_b$, identiques ou différents, représentent un atome d'hydrogène, un radical hydroxy, un radical alkyle renfermant de 1 à 4 atomes de carbone ou un radical alkoxy renfermant de 1 à 4 atomes de carbone, pour obtenir un composé de formule (VII) :

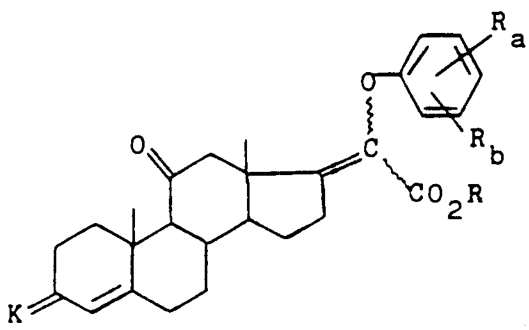

(VII)

dans laquelle K, R, $R_a$ et $R_b$ sont définis comme précédemment, que l'on soumet à l'action d'un agent réducteur, pour obtenir un composé de formule (VIII) :

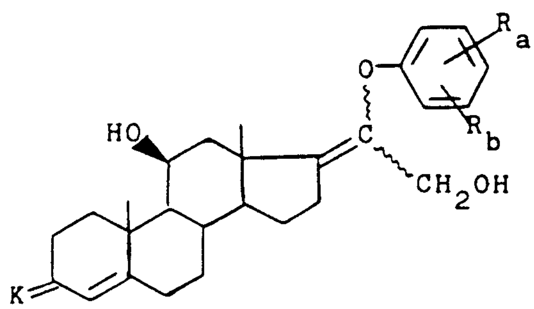

(VIII)

dans laquelle K, $R_a$ et $R_b$ sont définis comme précédemment, dont on déprotège la fonction 3-oxo pour obtenir un composé de formule (IX) :

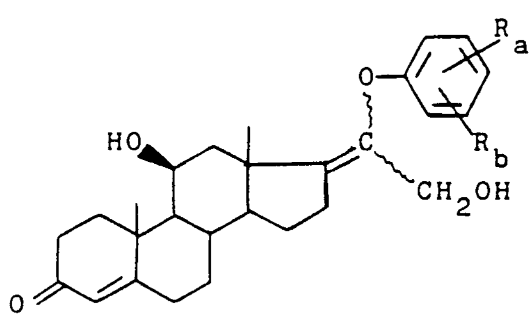

(IX)

dans laquelle $R_a$ et $R_b$ sont définis comme précédemment, que l'on traite par un agent d'époxydation, pour obtenir un composé de formule (X) :

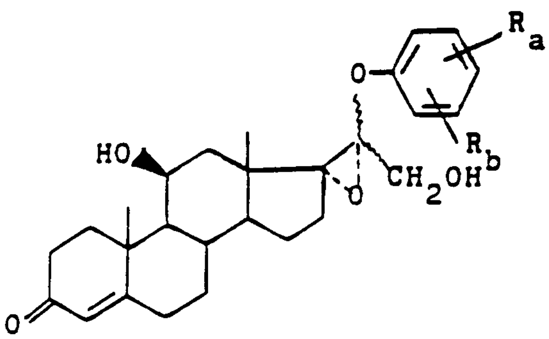

(X)

dans laquelle $R_a$ et $R_b$ sont définis comme précédemment, que l'on hydrolyse en milieu acide, pour obtenir le composé de formule (I) attendu.

**2.** Procédé de préparation de l'hydrocortisone selon la revendication 1, caractérise en ce que l'on soumet une halohydrine de formule (II) :

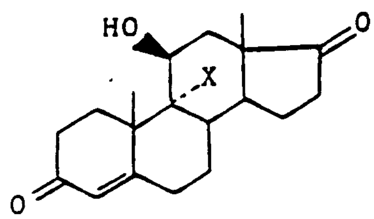

(II)

dans laquelle X est défini comme à la revendication 1, à une réaction de réarrangement en présence d'un alcool ou d'un polyalcool pour obtenir après traitement par un acide, le composé de formule (III) :

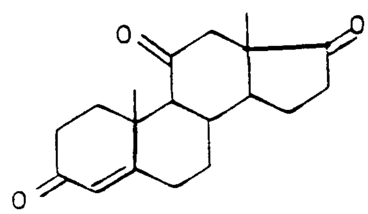

(III)

dont on protège sélectivement la fonction 3-oxo par action d'un thiol ou d'un dithiol de formule :

$HO-(CH_2)_n-SH$ ou $HS-(CH_2)_n-SH$

dans laquelle n est défini comme à la revendication 1, pour obtenir un composé de formule (IV) :

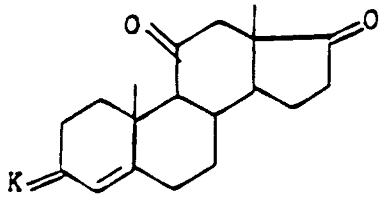

(IV)

dans laquelle K est défini comme à la revendication 1, que l'on traite par un réactif de formule $Hal_3C-CO_2R$ puis poursuit la synthèse comme décrit à la revendication 1.

3. Procédé de préparation de l'hydrocortisone selon la revendication 1, caractérisé en ce que l'on traite un composé de formule (II) telle que définie à la revendication 1, par un agent de protection sélectif de la fonction 3-oxo tel que défini à la revendication 1, pour obtenir un composé de formule (V) :

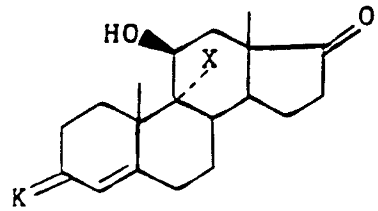

(V)

dans laquelle X et K sont définis comme à la revendication 1, que l'on soumet à une réaction de réarrangement en présence d'un alcool ou d'un polyalcool, pour obtenir après traitement par un acide, un composé de formule (IV) telle que définie à la revendication 1, puis traite ledit composé de formule (IV) par un réactif de formule :

$Hal_3C\text{-}CO_2R$

puis poursuit la synthèse comme décrit à la revendication 1.

4. Procédé selon la revendication 1, 2 ou 3, caractérisé en ce que dans le composé de formule (II), X représente un atome de brome.

5. Procédé selon la revendication 1, caractérisé en ce que le réarrangement de l'halohydrine est effectué en présence d'éthylène glycol, utilisé en excès.

6. Procédé selon l'une quelconque des revendications 1 à 5, caractérisé en ce que le réarrangement est effectué en présence d'un cosolvant de point d'ébullition inférieur à 100 °C, au reflux de celui-ci.

7. Procédé selon la revendication 6, caractérisé en ce que le cosolvant est l'acétate d'éthyle.

8. Procédé selon l'une quelconque des revendications 1 à 3, caractérisé en ce que l'on effectue le blocage sélectif de la fonction 3-oxo par l'éthane dithiol, en présence d'acide chlorhydrique ou bromhydrique concentré, en quantité catalytique, ou d'étherate de trifluorure de bore.

9. Procédé selon la revendication 1 ou 3, caractérisé en ce que l'on effectue le blocage de la fonction 3-oxo puis le réarrangement de l'halohydrine sans isolement de l'intermédiaire de formule (V).

10. Procédé selon l'une quelconque des revendications 1 à 3, caractérisé en ce que le trihaloacétate est un trichloroacétate de méthyle ou d'éthyle.

11. Procédé selon l'une quelconque des revendications 1 à 3 et 10, caractérisé en ce que l'acide de Lewis est le tétra-chlorure de titane.

12. Procédé selon l'une quelconque des revendications 1 à 3, caractérisé en ce que dans le phénol de formule

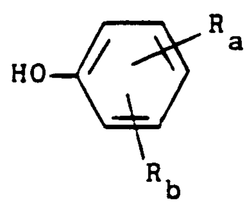

,

$R_a$ et $R_b$ représentent un atome d'hydrogène, un radical hydroxy ou un radical méthyle.

**13.** Procédé selon l'une quelconque des revendications 1 à 3, caractérisé en ce que l'agent réducteur est un hydrure d'aluminium ou un borohydrure alcalin.

**14.** Procédé selon l'une quelconque des revendications 1 à 3 et 8, caractérisé en ce que la déprotection de la fonction 3-oxo est réalisée par action soit de l'iode en présence d'une base, soit de l'iode en quantité catalytique, en présence d'un agent oxydant.

**15.** Procédé selon l'une quelconque des revendications 1 à 3, caractérisé en ce que l'agent d'époxydation est un peracide et l'agent d'hydrolyse est un acide minéral aqueux.

**16.** Les composés de formule (F) :

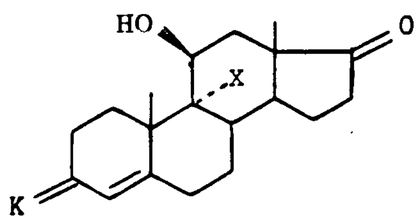

(F)

dans laquelle K représente un groupement protecteur du radical oxo de formule

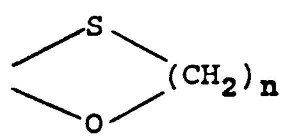

ou de préférence

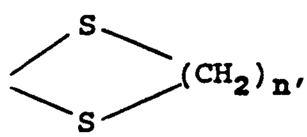

dans laquelle n est égal à 2 ou 3 et notamment égal à 2 et X représente un atome de chlore, de brome ou d'iode et notamment un atome de brome.

**17.** Les composés de formule (G) :

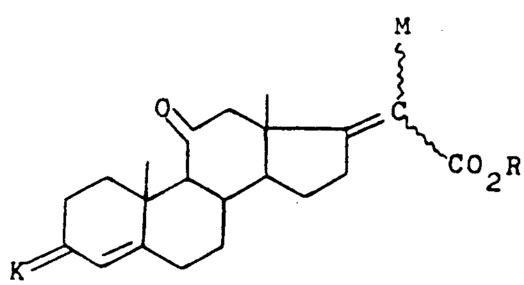

(G)

dans laquelle K représente un groupement protecteur du radical oxo de formule

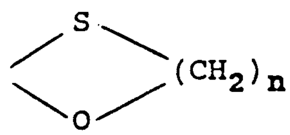

ou de préférence

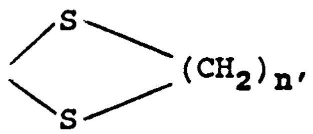

dans laquelle n est égal à 2 ou 3 et notamment égal à 2 et M représente soit un atome de chlore ou de brome et notamment un atome de chlore, soit un groupement

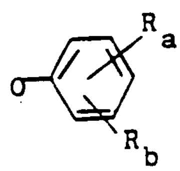

dans lequel $R_a$ et $R_b$ ont la signification indiquée à la revendication 1 et représentent notamment un atome d'hydrogène, un radical hydroxy ou un radical méthyle et R a la signification indiquée à la revendication 1 et notamment méthyle ou éthyle, étant étendu que lorsque R représente un reste silyle, il s'agit d'un reste trialkylsilyle, ou d'un reste triphénylsilyle ou diphényl tert-butylsilyle.

**18.** Les composés de formule (J) :

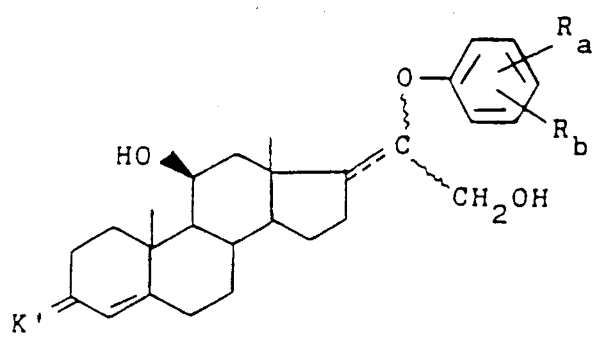

(J)

dans laquelle soit K' représente un atome d'oxygène ou un radical K, lequel représente un groupement protecteur du radical oxo de formule

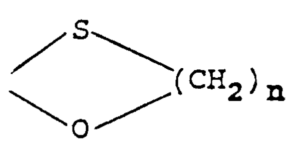

ou de préférence

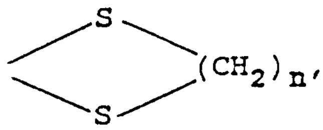

dans laquelle n est égal à 2 ou 3 et notamment égal à 2, le trait pointillé en position 17 représente une liaison, $R_a$ et $R_b$ ont la signification à la revendication 1 et représentent notamment un atome d'hydrogène, un radical hydroxy ou un radical méthyle, soit K' représente un atome d'oxygène, le trait pointillé en position 17 représente une fonction époxy, $R_a$ et $R_b$ ont la signification indiquée ci-dessus.

**Revendications pour l'Etat contractant suivant : ES**

1. Procédé de préparation de l'hydrocortisone de formule :

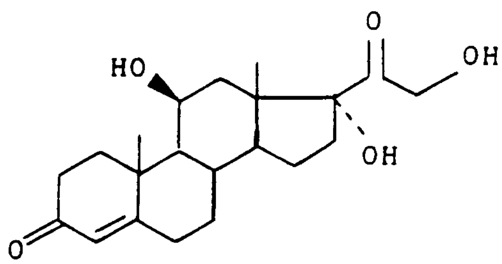

(I)

caractérisé en ce que ou bien l'on soumet une halohydrine de formule (II) :

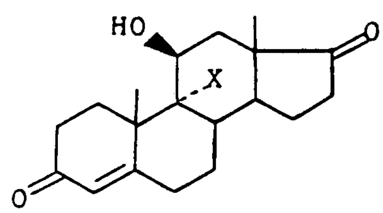

(II)

dans laquelle X représente un atome de chlore, de brome ou d'iode, à une réaction de réarrangement en présence d'un alcool ou d'un polyalcool, pour obtenir après traitement par un acide, le produit de formule (III) :

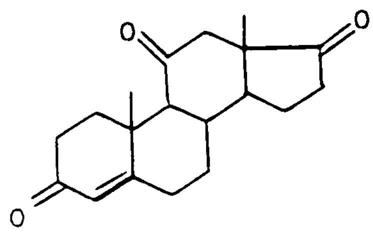

(III)

dont on protège sélectivement la fonction 3-oxo par action d'un thiol ou d'un dithiol de formule :

$HO-(CH_2)_n-SH$ ou $HS-(CH_2)_n-SH$

dans laquelle n est égal à 2 ou 3, pour obtenir un composé de formule (IV) :

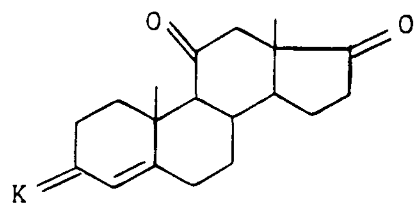

(IV)

dans laquelle K représente un groupement protecteur du radical 3-oxo de formule

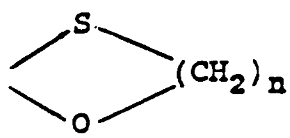

ou

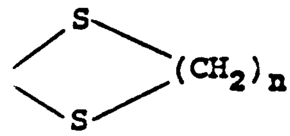

dans laquelle n est défini comme précédemment, ou bien l'on traite un composé de formule (II) telle que définie précédemment par un agent de blocage sélectif de la fonction 3-oxo tel que défini précédemment, pour obtenir un composé de formule (V) :

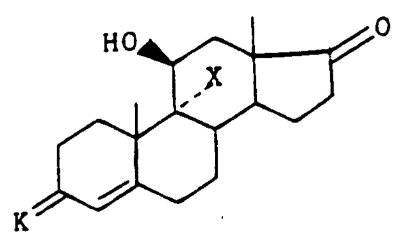

(V)

dans laquelle X et K sont définis comme précédemment, que l'on soumet à une réaction de réarrangement en présence d'un alcool ou d'un polyalcool, pour obtenir après traitement par un acide, un composé de formule (IV) telle que définie précédemment, puis traite ledit composé de formule (IV) par un trihaloacétate de formule

$Hal_3C\text{-}CO_2R$

dans laquelle Hal représente un atome de chlore ou de brome et R représente un radical alkyle renfermant de 1 à 6 atomes de carbone, un radical aralkyle renfermant de 7 à 15 atomes de carbone ou un reste silylé, en présence de zinc et d'un acide de Lewis, pour obtenir un composé de formule (VI) :

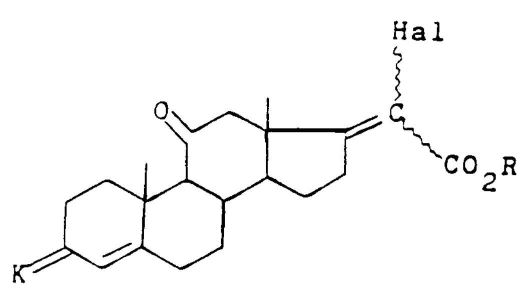

(VI)

dans laquelle K, Hal et R sont définis comme précédemment, que l'on traite, en milieu basique, par un phénol de formule :

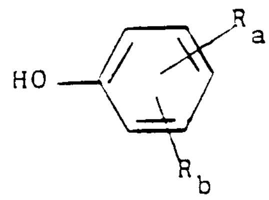

dans laquelle $R_a$ et $R_b$, identiques ou différents, représentent un atome d'hydrogène, un radical hydroxy, un radical alkyle renfermant de 1 à 4 atomes de carbone ou un radical alkoxy renfermant de 1 à 4 atomes de carbone, pour obtenir un composé de formule (VII) :

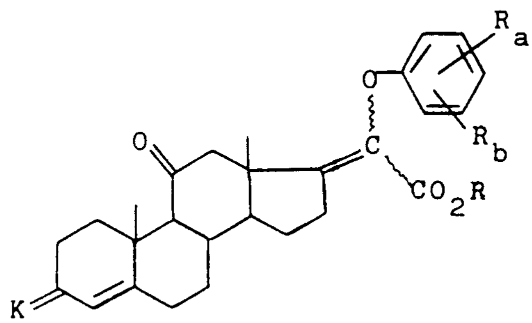

(VII)

dans laquelle K, R, $R_a$ et $R_b$ sont définis comme précédemment, que l'on soumet à l'action d'un agent réducteur, pour obtenir un composé de formule (VIII) :

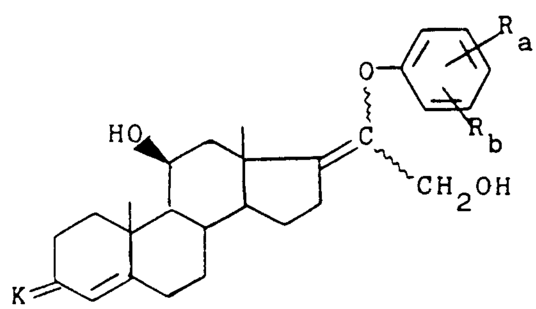

(VIII)

dans laquelle K, $R_a$ et $R_b$ sont définis comme précédemment, dont on déprotège la fonction 3-oxo pour obtenir un composé de formule (IX) :

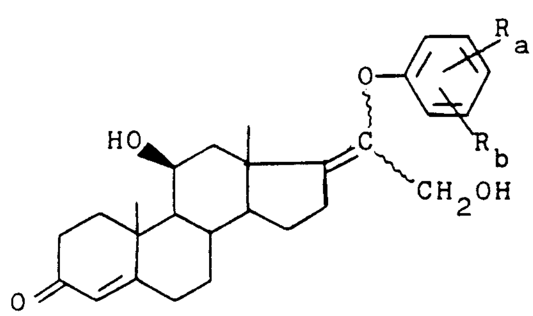

(IX)

dans laquelle $R_a$ et $R_b$ sont définis comme précédemment, que l'on traite par un agent d'époxydation, pour obtenir un composé de formule (X) :

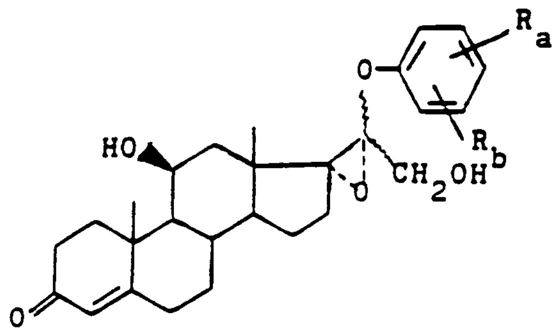

(X)

dans laquelle $R_a$ et $R_b$ sont définis comme précédemment, que l'on hydrolyse en milieu acide, pour obtenir le composé de formule (I) attendu.

**2.** Procédé de préparation de l'hydrocortisone selon la revendication 1, caractérisé en ce que l'on soumet une halohydrine de formule (II) :

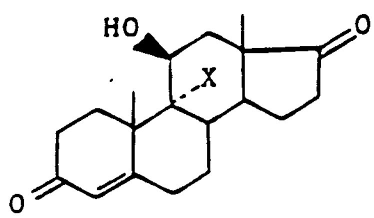

(II)

dans laquelle X est défini comme à la revendication 1, à une réaction de réarrangement en présence d'un alcool ou d'un polyalcool pour obtenir après traitement par un acide, le composé de formule (III) :

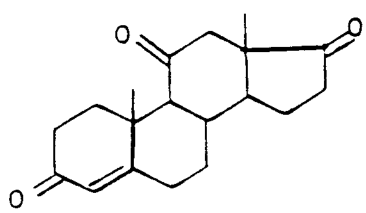

(III)

dont on protège sélectivement la fonction 3-oxo par action d'un thiol ou d'un dithiol de formule :

$HO-(CH_2)_n-SH$ ou $HS-(CH_2)_n-SH$

dans laquelle n est défini comme à la revendication 1, pour obtenir un composé de formule (IV) :

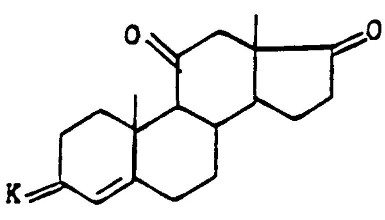

(IV)

dans laquelle K est défini comme à la revendication 1, que l'on traite par un réactif de formule $Hal_3C-CO_2R$ puis poursuit la synthèse comme décrit à la revendication 1.

3. Procédé de préparation de l'hydrocortisone selon la revendication 1, caractérisé en ce que l'on traite un composé de formule (II) telle que définie à la revendication 1, par un agent de protection sélectif de la fonction 3-oxo tel que défini à la revendication 1, pour obtenir un composé de formule (V) :

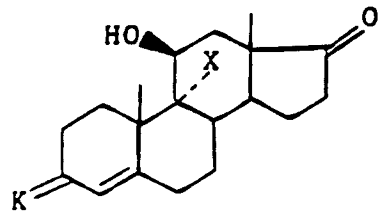

(V)

dans laquelle X et K sont définis comme à la revendication 1, que l'on soumet à une réaction de réarrangement en présence d'un alcool ou d'un polyalcool, pour obtenir après traitement par un acide, un composé de formule (IV) telle que définie à la revendication 1, puis traite ledit composé de formule (IV) par un réactif de formule :

$Hal_3C\text{-}CO_2R$

puis poursuit la synthèse comme décrit à la revendication 1.

4. Procédé selon la revendication 1, 2 ou 3, caractérisé en ce que dans le composé de formule (II), X représente un atome de brome.

5. Procédé selon la revendication 1, caractérisé en ce que le réarrangement de l'halohydrine est effectué en présence d'éthylène glycol, utilisé en excès.

6. Procédé selon l'une quelconque des revendications 1 à 5, caractérisé en ce que le réarrangement est effectué en présence d'un cosolvant de point d'ébullition inférieur à 100 ° C, au reflux de celui-ci.

7. Procédé selon la revendication 6, caractérisé en ce que le cosolvant est l'acétate d'éthyle.

8. Procédé selon l'une quelconque des revendications 1 à 3, caractérisé en ce que l'on effectue le blocage sélectif de la fonction 3-oxo par l'éthane dithiol, en présence d'acide chlorhydrique ou bromhydrique concentré, en quantité catalytique, ou d'étherate de trifluorure de bore.

9. Procédé selon la revendication 1 ou 3, caractérisé en ce que l'on effectue le blocage de la fonction 3-oxo puis le réarrangement de l'halohydrine sans isolement de l'intermédiaire de formule (V).

10. Procédé selon l'une quelconque des revendications 1 à 3, caractérisé en ce que le trihaloacétate est un trichloroacétate de méthyle ou d'éthyle.

11. Procédé selon l'une quelconque des revendications 1 à 3 et 10, caractérisé en ce que l'acide de Lewis est le tétrachlorure de titane.

12. Procédé selon l'une quelconque des revendications 1 à 3, caractérisé en ce que dans le phénol de formule

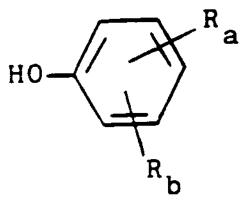

,

$R_a$ et $R_b$ représentent un atome d'hydrogène, un radical hydroxy ou un radical méthyle.

**13.** Procédé selon l'une quelconque des revendications 1 à 3, caractérisé en ce que l'agent réducteur est un hydrure d'aluminium ou un borohydrure alcalin.

**14.** Procédé selon l'une quelconque des revendications 1 à 3 et 8, caractérisé en ce que la déprotection de la fonction 3-oxo est réalisée par action soit de l'iode en présence d'une base, soit de l'iode en quantité catalytique, en présence d'un agent oxydant.

**15.** Procédé selon l'une quelconque des revendications 1 à 3, caractérisé en ce que l'agent d'époxydation est un peracide et l'agent d'hydrolyse est un acide minéral aqueux.

**Claims**

**Claims for the following Contracting States : AT, BE, CH, LI, DE, DK, FR, GB, GR, IT, LU, NL, PT, SE, IE**

**1.** Preparation process for hydrocortisone of formula:

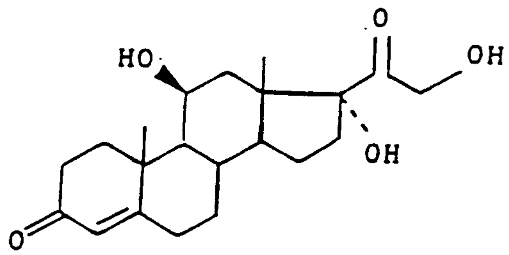

(I)

characterized in that either a halohydrin of formula (II):

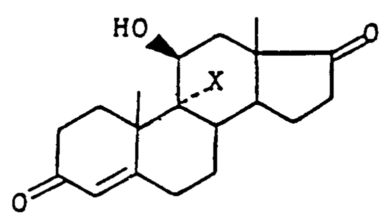

(II)

in which X represents a chlorine, bromine or iodine atom, is subjected to a rearrangement reaction in the presence of an alcohol or a polyalcohol, in order to obtain, after treatment with an acid, the product of formula (III):

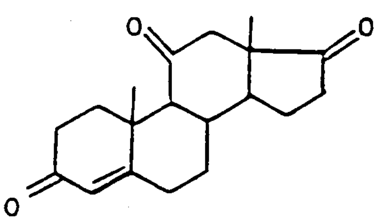

(III)

the 3-oxo function of which is selectively protected by the action of a thiol or a dithiol of formula:

$HO-(CH_2)_n-SH$ or $HS-(CH_2)_n-SH$

in which n is equal to 2 or 3, in order to obtain a compound of formula (IV):

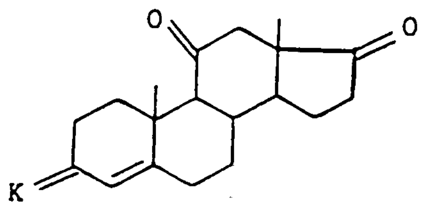

(IV)

in which K represents a protector group of the 3-oxo radical of formula

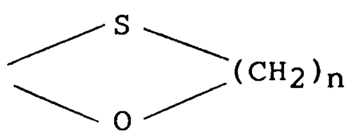

or

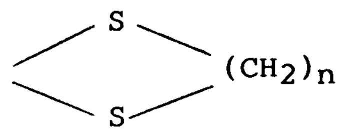

in which n is defined as previously, or a compound of formula (II) as defined previously is treated with a selective blocking agent of the 3-oxo function as defined previously, in order to obtain a compound of formula (V):

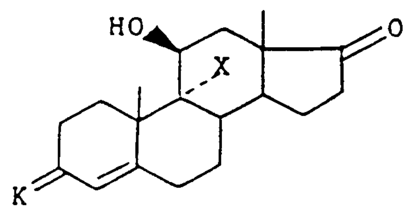

(V)

in which X and K are defined as previously, which is subjected to a rearrangement reaction in the presence of an alcohol or a polyalcohol, in order to obtain, after treatment with an acid, a compound of formula (IV) as defined previously, then said compound of formula (IV) is treated with a trihaloacetate of formula

$Hal_3C-CO_2R$

in which Hal represents a chlorine or bromine atom and R represents an alkyl radical containing 1 to 6 carbon atoms, an aralkyl radical containing 7 to 15 carbon atoms or a silylated remainder, in the presence of zinc and of a Lewis acid, in order to obtain a compound of formula (VI):

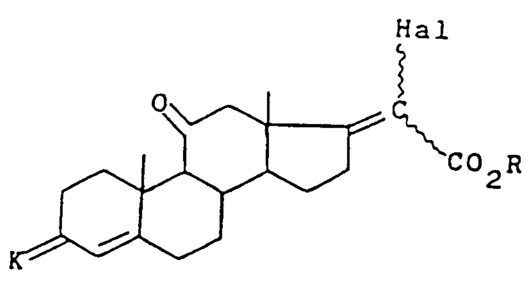

(VI)

in which K, Hal and R are defined as previously, which is treated, in a basic medium, by a phenol of formula:

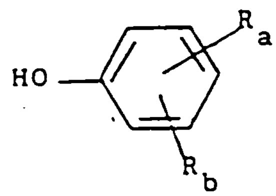

in which $R_a$ and $R_b$, identical or different, represent a hydrogen atom, a hydroxy radical, an alkyl radical containing 1 to 4 carbon atoms or an alkoxy radical containing 1 to 4 carbon atoms, in order to obtain a compound of formula (VII):

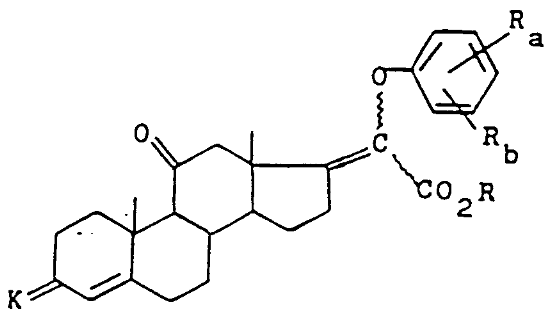

(VII)

in which K, R, $R_a$ and $R_b$ are defined as previously, which is subjected to the action of a reducing agent, in order to obtain a compound of formula (VIII):

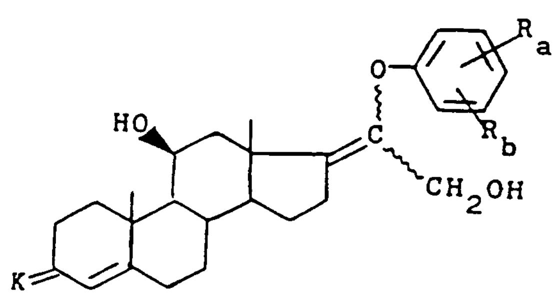

(VIII)

in which K, $R_a$ and $R_b$ are defined as previously, the 3-oxo function of which is deprotected in order to obtain a compound of formula (IX):

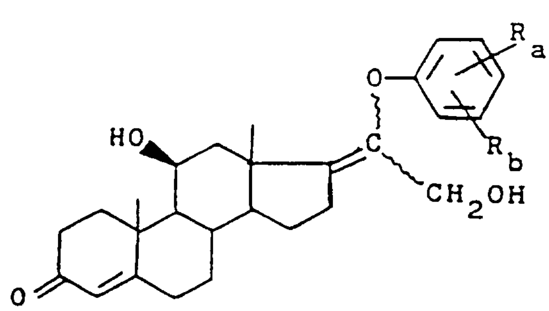

(IX)

in which $R_a$ and $R_b$ are defined as previously, which is treated with an epoxidation agent, in order to obtain a compound of formula (X):

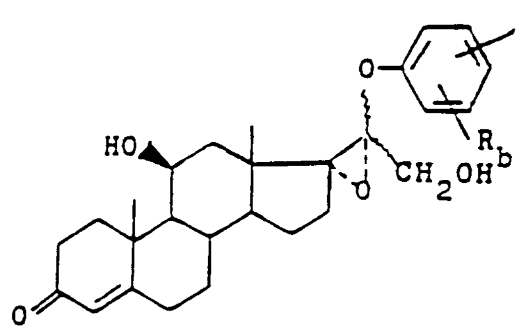

(X)

in which $R_a$ and $R_b$ are defined as previously, which is hydrolyzed in an acid medium, in order to obtain the expected compound of formula (I).

**2.** Preparation process for hydrocortisone according to claim 1, characterized in that a halohydrin of formula (II):

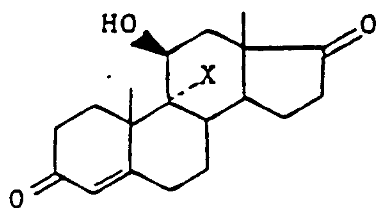

(II)

in which X is defined as in claim 1, is subjected to a rearrangement reaction in the presence of an alcohol or a polyalcohol in order to obtain, after treatment with an acid, the compound of formula (III):

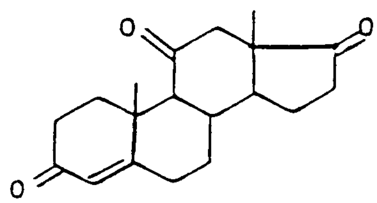

(III)

the 3-oxo function of which is selectively protected by the action of a thiol or a dithiol of formula:

$HO-(CH_2)_n-SH$ or $HS-(CH_2)_n-SH$

in which n is defined as in claim 1, in order to obtain a compound of formula (IV):

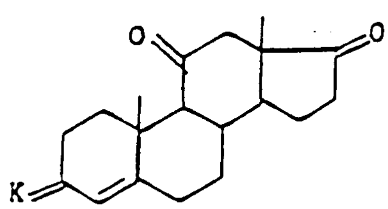

(IV)

in which K is defined as in claim 1, which is treated with a reagent of formula $Hal_3C-CO_2R$ then the

synthesis is continued as described in claim 1.

**3.** Preparation process for hydrocortisone according to claim 1, characterized in that a compound of formula (II) as defined in claim 1 is treated with a selective protection agent of the 3-oxo function as defined in claim 1, in order to obtain a compound of formula (V):

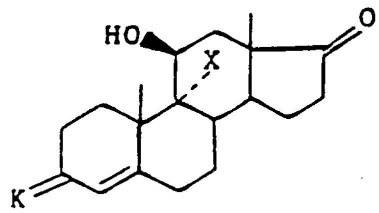

(V)

in which X and K are defined as in claim 1, which is subjected to a rearrangement reaction in the presence of an alcohol or a polyalcohol, in order to obtain, after treatment with an acid, a compound of formula (IV) as defined in claim 1, then said compound of formula (IV) is treated with a reagent of formula:

$Hal_3C\text{-}CO_2R$

then the synthesis is continued as described in claim 1.

**4.** Process according to claim 1, 2 or 3, characterized in that in the compound of formula (II), X represents a bromine atom.

**5.** Process according to claim 1, characterized in that the rearrangement of the halohydrin is carried out in the presence of ethylene glycol, used in excess.

**6.** Process according to any one of claims 1 to 5, characterized in that the rearrangement is carried out in the presence of a cosolvent with a boiling point of less than 100 °C, under reflux of the latter.

**7.** Process according to claim 6, characterized in that the cosolvent is ethyl acetate.

**8.** Process according to any one of claims 1 to 3, characterized in that the selective blocking of the 3-oxo function is carried out by ethane dithiol, in the presence of concentrated hydrobromic or hydrochloric acid, in catalytic quantity, or boron trifluoride etherate.

**9.** Process according to claim 1 or 3, characterized in that the blocking of the 3-oxo function then the rearrangement of the halohydrin is carried out without isolation of the intermediate of formula (V).

**10.** Process according to any one of claims 1 to 3, characterized in that the trihaloacetate is a methyl or ethyl trichloroacetate.

**11.** Process according to any one of claims 1 to 3 and 10, characterized in that the Lewis acid is titanium tetrachloride.

**12.** Process according to any one of claims 1 to 3, characterized in that in the phenol of formula

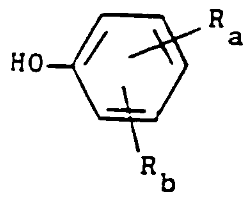

,

$R_a$ and $R_b$ represent a hydrogen atom, a hydroxy radical or a methyl radical.

**13.** Process according to any one of claims 1 to 3, characterized in that the reducing agent is an aluminium hydride or an alkaline borohydride.

**14.** Process according to any one of claims 1 to 3 and 8, characterized in that the deprotection of the 3-oxo function is carried out by the action either of iodine in the presence of a base, or of iodine in catalytic quantity, in the presence of an oxidizing agent.

**15.** Process according to any one of claims 1 to 3, characterized in that the epoxidation agent is a peracid and the hydrolysis agent is an aqueous mineral acid.

**16.** The compounds of formula (F):

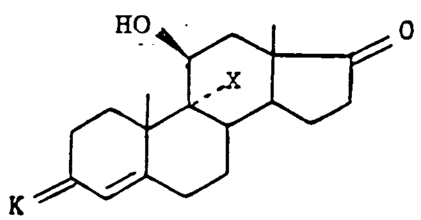

(F)

in which K represents a protector group of the oxo radical of formula

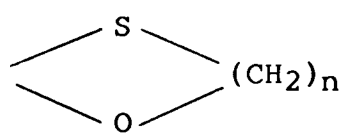

or preferably

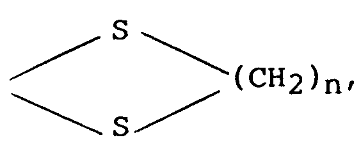

in which n is equal to 2 or 3 and in particular equal to 2 and X represents a chlorine, bromine or iodine atom and in particular a bromine atom.

**17.** The compounds of formula (G):

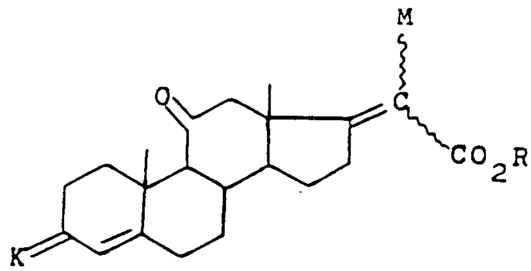

(G)

in which K represents a protector group of the oxo radical of formula

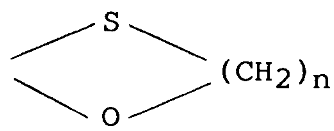

or preferably

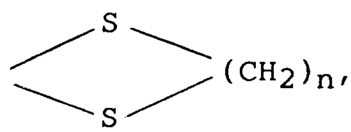

in which n is equal to 2 or 3 and in particular equal to 2 and M represents either a chlorine or bromine atom and in particular a chlorine atom, or a

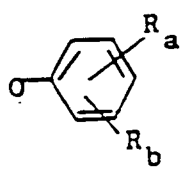

group in which $R_a$ and $R_b$ have the meaning indicated in claim 1 and represent in particular a hydrogen atom, a hydroxy radical or a methyl radical and R has the meaning indicated in claim 1 and in particular methyl or ethyl, it being understood that when R represents a silyl remainder, it is a trialkylsilyl remainder or a triphenylsilyl or diphenyl tert-butylsilyl remainder.

**18.** The compounds of formula (J):

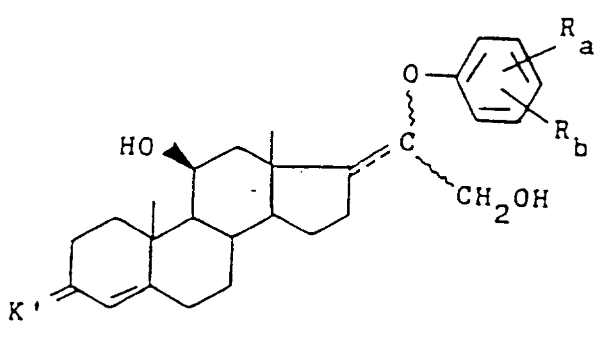

(J)

in which either K' represents an oxygen atom or a radical K, which represents a protector group of the

oxo radical of formula

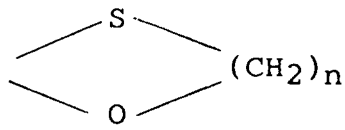

or preferably

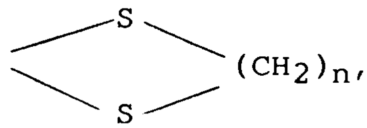

in which n is equal to 2 or 3 and in particular equal to 2, the dotted line in position 17 represents a bond, $R_a$ and $R_b$ have the meaning in claim 1 and represent in particular a hydrogen atom, a hydroxy radical or a methyl radical, or K' represents an oxygen atom, the dotted line in position 17 represents an epoxy function, $R_a$ and $R_b$ have the meaning indicated above.

**Claims for the following Contracting State : ES**

**1.** Preparation process for hydrocortisone of formula:

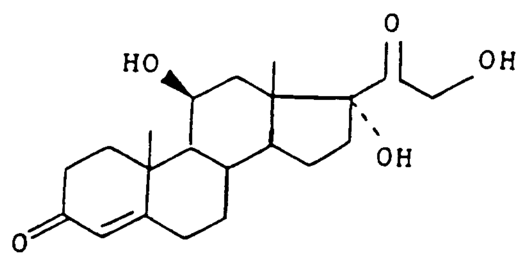

(I)

characterized in that either a halohydrin of formula (II):

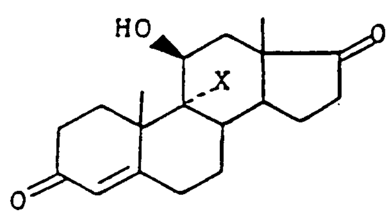

(II)

in which X represents a chlorine, bromine or iodine atom, is subjected to a rearrangement reaction in the presence of an alcohol or a polyalcohol, in order to obtain, after treatment with an acid, the product of formula (III):

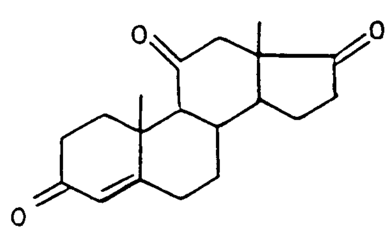

(III)

the 3-oxo function of which is selectively protected by the action of a thiol or a dithiol of formula:

$HO-(CH_2)_n-SH$ or $HS-(CH_2)_n-SH$

in which n is equal to 2 or 3, in order to obtain a compound of formula (IV):

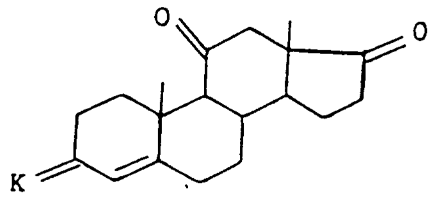

(IV)

in which K represents a protector group of the 3-oxo radical of formula

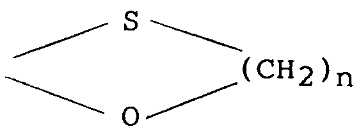

or

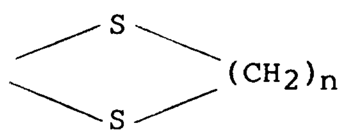

in which n is defined as previously, or a compound of formula (II) as defined previously is treated with a selective blocking agent of the 3-oxo function as defined previously, in order to obtain a compound of formula (V):

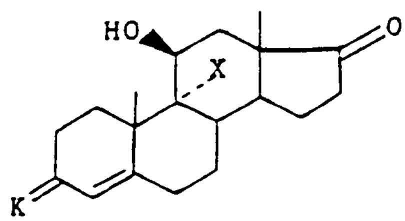

(V)

in which X and K are defined as previously, which is subjected to a rearrangement reaction in the presence of an alcohol or a polyalcohol, in order to obtain, after treatment with an acid, a compound of formula (IV) as defined previously, then said compound of formula (IV) is treated with a trihaloacetate of formula

$Hal_3C-CO_2R$

in which Hal represents a chlorine or bromine atom and R represents an alkyl radical containing 1 to 6 carbon atoms, an aralkyl radical containing 7 to 15 carbon atoms or a silylated remainder, in the presence of zinc and of a Lewis acid, in order to obtain a compound of formula (VI):

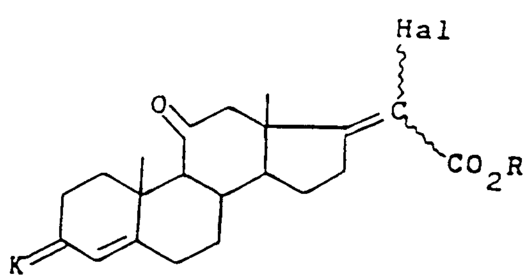

(VI)

in which K, Hal and R are defined as previously, which is treated, in a basic medium, by a phenol of

formula:

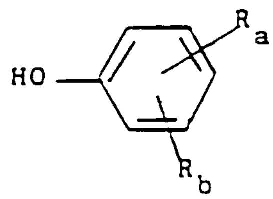

in which $R_a$ and $R_b$, identical or different, represent a hydrogen atom, a hydroxy radical, an alkyl radical containing 1 to 4 carbon atoms or an alkoxy radical containing 1 to 4 carbon atoms, in order to obtain a compound of formula (VII):

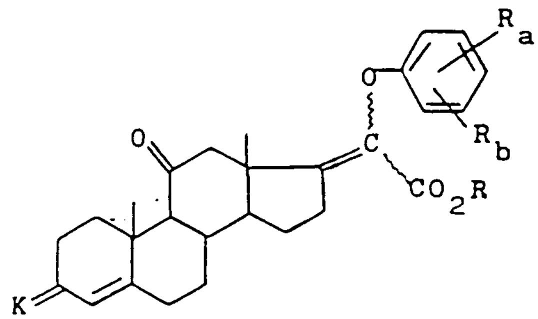

(VII)

in which K, R, $R_a$ and $R_b$ are defined as previously, which is subjected to the action of a reducing agent, in order to obtain a compound of formula (VIII):

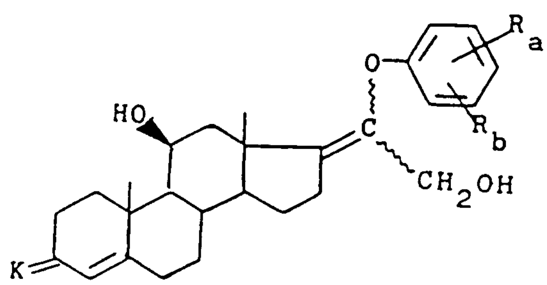

(VIII)

in which K, $R_a$ and $R_b$ are defined as previously, the 3-oxo function of which is deprotected in order to obtain a compound of formula (IX):

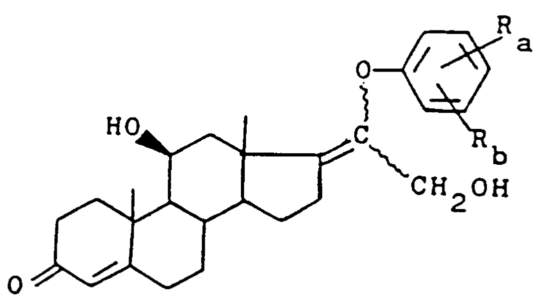

(IX)

in which $R_a$ and $R_b$ are defined as previously, which is treated with an epoxidation agent, in order to obtain a compound of formula (X):

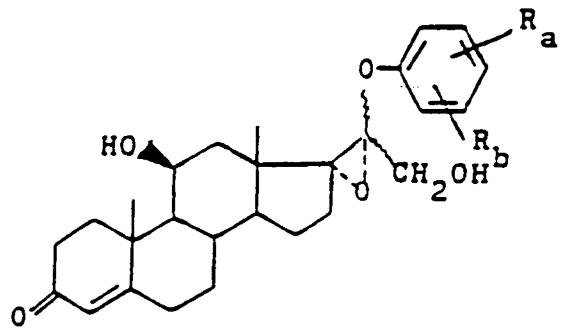

(X)

in which $R_a$ and $R_b$ are defined as previously, which is hydrolyzed in an acid medium, in order to obtain the expected compound of formula (I).

**2.** Preparation process for hydrocortisone according to claim 1, characterized in that a halohydrin of formula (II):

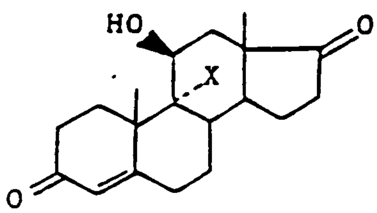

(II)

in which X is defined as in claim 1, is subjected to a rearrangement reaction in the presence of an alcohol or a polyalcohol in order to obtain, after treatment with an acid, the compound of formula (III):

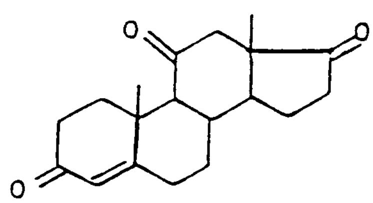

(III)

the 3-oxo function of which is selectively protected by the action of a thiol or a dithiol of formula:

$HO-(CH_2)_n-SH$ or $HS-(CH_2)_n-SH$

in which n is defined as in claim 1, in order to obtain a compound of formula (IV):

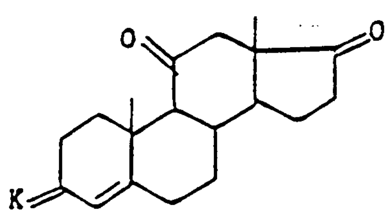

(IV)

in which K is defined as in claim 1, which is treated with a reagent of formula $Hal_3C-CO_2R$ then the synthesis is continued as described in claim 1.

3. Preparation process for hydrocortisone according to claim 1, characterized in that a compound of formula (II) as defined in claim 1 is treated with a selective protection agent of the 3-oxo function as defined in claim 1, in order to obtain a compound of formula (V):

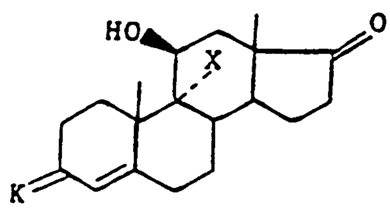

(V)

in which X and K are defined as in claim 1, which is subjected to a rearrangement reaction in the presence of an alcohol or a polyalcohol, in order to obtain, after treatment with an acid, a compound of formula (IV) as defined in claim 1, then said compound of formula (IV) is treated with a reagent of formula:

$Hal_3C\text{-}CO_2R$

then the synthesis is continued as described in claim 1.

4. Process according to claim 1, 2 or 3, characterized in that in the compound of formula (II), X represents a bromine atom.

5. Process according to claim 1, characterized in that the rearrangement of the halohydrin is carried out in the presence of ethylene glycol, used in excess.

6. Process according to any one of claims 1 to 5, characterized in that the rearrangement is carried out in the presence of a cosolvent with a boiling point of less than 100 ° C, under reflux of the latter.

7. Process according to claim 6, characterized in that the cosolvent is ethyl acetate.

8. Process according to any one of claims 1 to 3, characterized in that the selective blocking of the 3-oxo function is carried out by ethane dithiol, in the presence of concentrated hydrobromic or hydrochloric acid, in catalytic quantity, or boron trifluoride etherate.

9. Process according to claim 1 or 3, characterized in that the blocking of the 3-oxo function then the rearrangement of the halohydrin is carried out without isolation of the intermediate of formula (V).

10. Process according to any one of claims 1 to 3, characterized in that the trihaloacetate is a methyl or ethyl trichloroacetate.

11. Process according to any one of claims 1 to 3 and 10, characterized in that the Lewis acid is titanium tetrachloride.

12. Process according to any one of claims 1 to 3, characterized in that in the phenol of formula

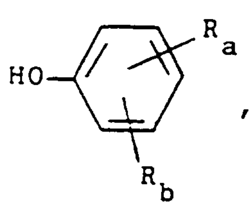

,

$R_a$ and $R_b$ represent a hydrogen atom, a hydroxy radical or a methyl radical.

13. Process according to any one of claims 1 to 3, characterized in that the reducing agent is an aluminium hydride or an alkaline borohydride.

14. Process according to any one of claims 1 to 3 and 8, characterized in that the deprotection of the 3-oxo function is carried out by the action either of iodine in the presence of a base, or of iodine in catalytic quantity, in the presence of an oxidizing agent.

15. Process according to any one of claims 1 to 3, characterized in that the epoxidation agent is a peracid and the hydrolysis agent is an aqueous mineral acid.

**Patentansprüche**
**Patentansprüche für folgende Vertragsstaaten : AT, BE, CH, LI, DE, DK, FR, GB, GR, IT, LU, NL, PT, SE, IE**

1. Verfahren zur Herstellung von Hydrocortison der Formel (I)

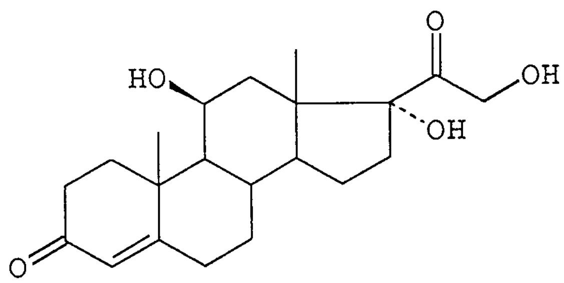

(I)

dadurch gekennzeichnet, daß man entweder ein Halohydrin der Formel (II)

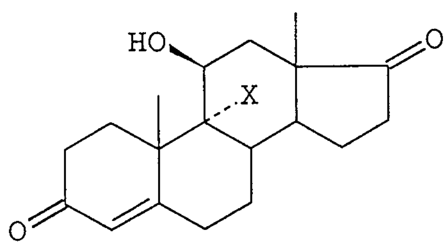

(II)

in der X ein Chlor-, Brom- oder Iodatom darstellt, einer Umlagerungsreaktion in Anwesenheit eines Alkohols oder eines Polyalkohols unterzieht, um nach Behandlung durch eine Säure das Produkt der Formel (III)

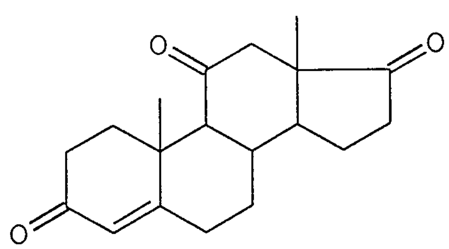

(III)

zu erhalten, bei dem man selektiv die Funktion 3-Oxo durch Einwirkung eines Thiols oder eines Dithiols der Formel

$HO-(CH_2)_n-SH$ oder $HS-(CH_2)_n-SH$

schützt, in der n gleich 2 oder 3 ist, um eine Verbindung der Formel (IV)

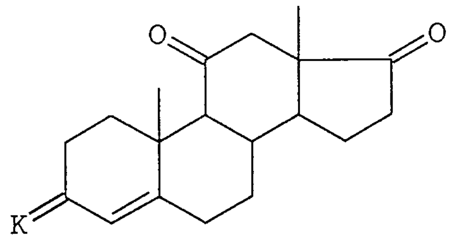

(IV)

zu erhalten, in der K eine Schutzgruppe der Formel

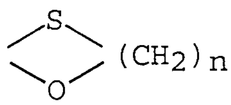

oder

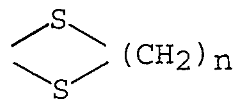

in der n wie vorstehend definiert ist, für den Rest 3-Oxo darstellt,
oder eine wie vorstehend definierte Verbindung der Formel (II) durch ein selektives Blockierungsmittel für die wie vorstehend definierte Funktion 3-Oxo behandelt, um eine Verbindung der Formel (V)

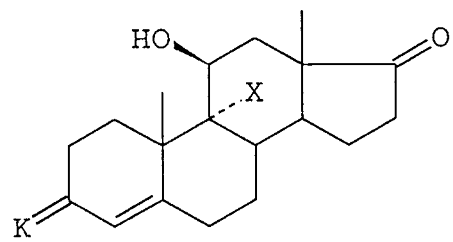

(V)

zu erhalten, in der X und K wie vorstehend definiert sind, die man einer Umlagerungsreaktion in Anwesenheit eines Alkohols oder eines Polyalkohols unterzieht, um nach Behandlung durch eine Säure eine wie vorstehend definierte Verbindung der Formel (IV) zu erhalten, wonach man die genannte Verbindung der Formel (IV) mit einem Trihaloacetat der Formel

$Hal_3C\text{-}CO_2R$

in der Hal ein Chlor- oder Bromatom darstellt und R einen Alkylrest mit 1 bis 6 Kohlenstoffatomen, einen Aralkylrest mit 7 bis 15 Kohlenstoffatomen oder einen Silylrest bedeutet, in Anwesenheit von Zink und einer Lewis-Säure behandelt, um eine Verbindung der Formel (VI)

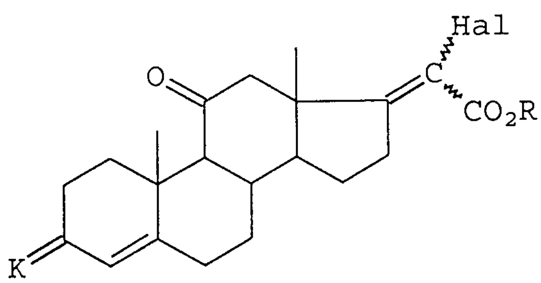

(VI)

zu erhalten, in der K, Hal und R wie vorstehend definiert sind, die man im basischen Medium mit einem Phenol der Formel

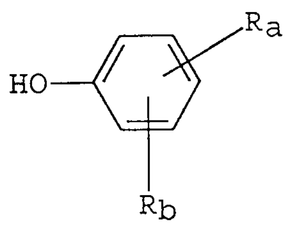

behandelt, in der $R_a$ und $R_b$, gleich oder verschieden, ein Wasserstoffatom, einen Hydroxyrest, einen Alkylrest mit 1 bis 4 Kohlenstoffatomen oder einen Alkoxyrest mit 1 bis 4 Kohlenstoffatomen darstellen, um eine Verbindung der Formel (VII)

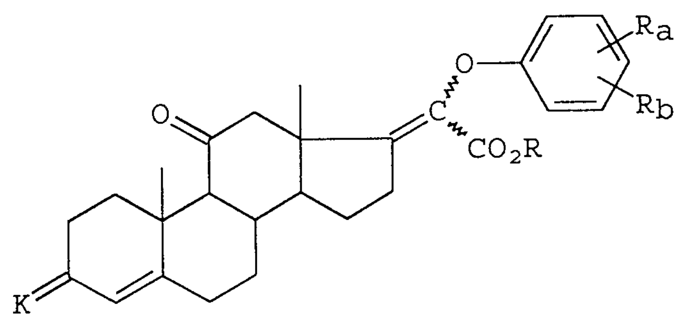

(VII)

zu erhalten, in der K, R, $R_a$ und $R_b$ wie vorstehend definiert sind, das man der Einwirkung eines Reduktionsmittels unterzieht, um eine Verbindung der Formel (VIII)

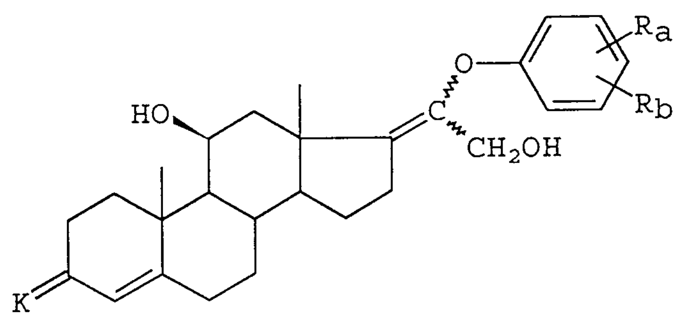

(VIII)

zu erhalten, in der K, $R_a$ und $R_b$ wie vorstehend definiert sind, bei der man von der Funktion 3-Oxo die Schutzgruppe entfernt, um eine Verbindung der Formel (IX)

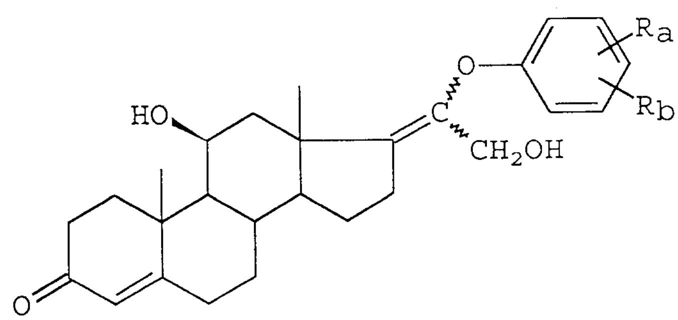

(IX)

zu erhalten, in der $R_a$ und $R_b$ wie vorstehend definiert sind, die man mit einem Epoxidierungsmittel behandelt, um eine Verbindung der Formel (X)

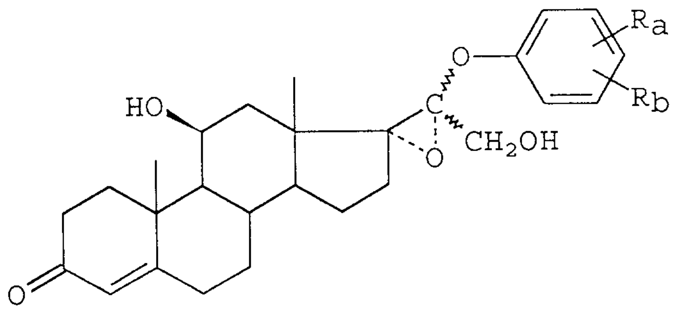

(X)

zu erhalten, in der $R_a$ und $R_b$ wie vorstehend definiert sind, die man dann im sauren Medium hydrolysiert, um die erwartete Verbindung der Formel (I) zu erhalten.

**2.** Verfahren zur Herstellung von Hydrocortison nach Anspruch 1, dadurch gekennzeichnet, daß man ein Halohydrin der Formel (II)

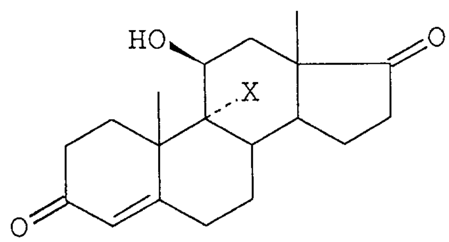

(II)

in der X wie in Anspruch 1 definiert ist, einer Umlagerungsreaktion in Anwesenheit eines Alkohols oder eines Polyalkohols unterzieht, um nach Behandlung durch eine Säure die Verbindung der Formel (III)

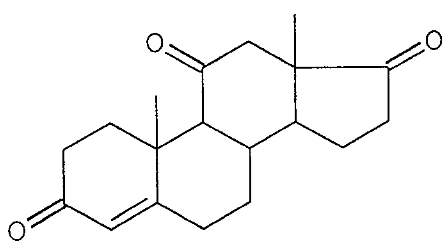

(III)

zu erhalten, bei der man selektiv die Funktion 3-Oxo durch Einwirkung eines Thiols oder eines Dithiols der Formel

$HO-(CH_2)_n-SH$ oder $HS-(CH_2)_n-SH$

schützt, in der n wie in Anspruch 1 definiert ist, um eine Verbindung der Formel (IV)

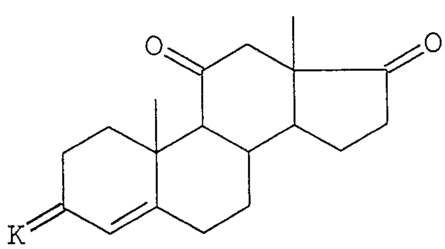

(IV)

zu erhalten, in der K wie in Anspruch 1 definiert ist, die man mit einem Reaktionspartner der Formel

$Hal_3C-CO_2R$

behandelt und anschließend die Synthese wie in Anspruch 1 beschrieben fortsetzt.

**3.** Verfahren zur Herstellung von Hydrocortison nach Anspruch 1, dadurch gekennzeichnet, daß man eine wie in Anspruch 1 definierte Verbindung der Formel (II) durch ein selektives Schutzmittel für die wie in Anspruch 1 definierte Funktion 3-Oxo behandelt, um eine Verbindung der Formel (V)

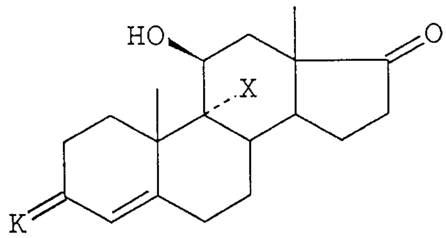

(V)

zu erhalten, in der X und K wie in Anspruch 1 definiert sind, die man einer Umlagerungsreaktion in Anwesenheit eines Alkohols oder eines Polyalkohols unterzieht, um nach Behandlung durch eine Säure eine wie in Anspruch 1 definierte Verbindung der Formel (IV) zu erhalten, wonach man die genannte Verbindung der Formel (IV) mit einem Reaktionspartner der Formel

$Hal_3C\text{-}CO_2R$

behandelt und anschließend die Synthese wie in Anspruch 1 beschrieben fortsetzt.

**4.** Verfahren nach Anspruch 1, 2 oder 3, dadurch gekennzeichnet, daß in der Verbindung der Formel (II) X ein Bromatom darstellt.

**5.** Verfahren nach Anspruch 1, dadurch gekennzeichnet, daß die Umlagerung des Halohydrins in Anwesenheit von Ethylenglycol, angewendet im Überschuß, durchgeführt wird.

**6.** Verfahren nach irgendeinem der Ansprüche 1 bis 5, dadurch gekennzeichnet, daß die Umlagerung in Anwesenheit eines Co-Lösungsmittels mit einem Siedepunkt von unter 100 °C, unter dessen Rückfluß durchgeführt wird.

**7.** Verfahren nach Anspruch 6, dadurch gekennzeichnet, daß das Co-Lösungsmittel Ethylacetat ist.

**8.** Verfahren nach irgendeinem der Ansprüche 1 bis 3, dadurch gekennzeichnet, daß man die selektive Blockierung der Funktion 3-Oxo mit Hilfe von Ethandithiol in Anwesenheit von konzentrierter Chlorwasserstoff- oder Bromwasserstoffsäure in katalytischer Menge oder von Bortrifluorid-etherat durchführt.

**9.** Verfahren nach Anspruch 1 oder 3, dadurch gekennzeichnet, daß man die Blockierung der Funktion 3-Oxo und die anschließende Umlagerung des Halohydrins ohne Isolierung des Zwischenproduktes der Formel (V) durchführt.

**10.** Verfahren nach irgendeinem der Ansprüche 1 bis 3, dadurch gekennzeichnet, daß das Trihaloacetat ein Trichloracetat von Methyl oder Ethyl ist.

**11.** Verfahren nach irgendeinem der Ansprüche 1 bis 3 und 10, dadurch gekennzeichnet, daß die Lewis-Säure Titantetrachlorid ist.

**12.** Verfahren nach irgendeinem der Ansprüche 1 bis 3, dadurch gekennzeichnet, daß in dem Phenol der Formel

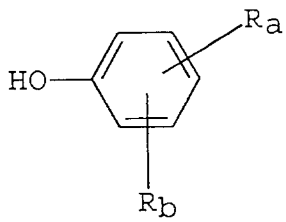

$R_a$ und $R_b$ ein Wasserstoffatom, einen Hydroxyrest oder einen Methylrest darstellen.

**13.** Verfahren nach irgendeinem der Ansprüche 1 bis 3, dadurch gekennzeichnet, daß das Reduktionsmittel ein Aluminiumhydrid oder ein Alkaliborhydrid ist.

**14.** Verfahren nach irgendeinem der Ansprüche 1 bis 3 und 8, dadurch gekennzeichnet, daß die Abspaltung der Schutzgruppe von der Funktion 3-Oxo entweder durch Einwirkung von Iod in Anwesenheit einer Base oder von Iod in katalytischer Menge in Anwesenheit eines Oxidationsmittels realisiert wird.

**15.** Verfahren nach irgendeinem der Ansprüche 1 bis 3, dadurch gekennzeichnet, daß das Epoxidierungsmittel eine Persäure und das Hydrolysemittel eine wäßrige Mineralsäure ist.

**16.** Die Verbindungen der Formel (F)

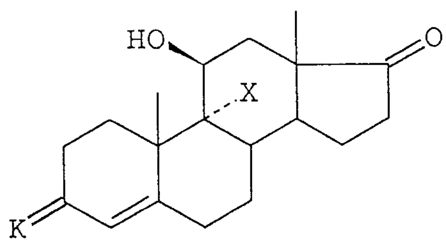

(F)

in der K eine Schutzgruppe der Formel

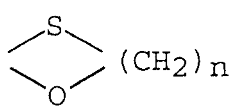

oder vorzugsweise

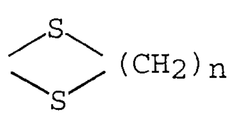

für den Rest 3-Oxo darstellt, wobei in der genannten Formel n gleich 2 oder 3 und insbesondere gleich 2 ist und X ein Chlor-, Brom- oder Iodatom und insbesondere ein Bromatom bedeutet.

**17.** Die Verbindungen der Formel (G)

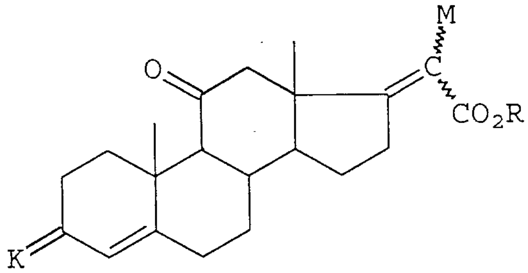

(G)

in der K eine Schutzgruppe der Formel

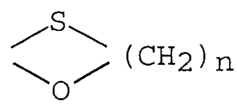

oder vorzugsweise

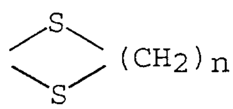

für den Rest 3-Oxo darstellt, wobei in der genannten Formel n gleich 2 oder 3 und insbesondere gleich 2 ist und M entweder ein Chlor- oder Bromatom und insbesondere ein Chloratom oder eine Gruppe

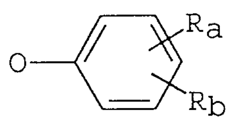

darstellt, in der $R_a$ und $R_b$ die in Anspruch 1 angegebene Bedeutung besitzen und insbesondere ein Wasserstoffatom, einen Hydroxyrest oder einen Methylrest darstellen und R die in Anspruch 1 angegebene Bedeutung aufweist und insbesondere Methyl oder Ethyl ist, mit der Maßgabe, daß es sich in dem Fall, wo R einen Silylrest bedeutet, um einen Trialkylsilylrest oder einen Triphenylsilylrest oder einen Diphenyl-tert.-butylsilylrest handelt.

**18.** Die Verbindungen der Formel (J)

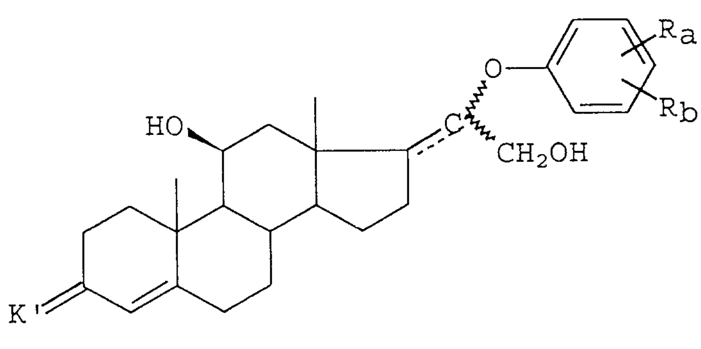

(J)

in der K' entweder ein Sauerstoffatom oder einen Rest K darstellt, der eine Schutzgruppe der Formel

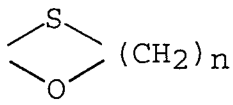

oder vorzugsweise

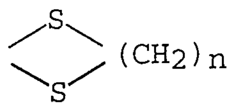

für den Rest 3-Oxo bedeutet, wobei in der genannten Formel n gleich 2 oder 3 und insbesondere gleich 2 ist, die punktierte Linie in Position 17 eine Bindung darstellt, $R_a$ und $R_b$ die Bedeutung von Anspruch 1 besitzen und insbesondere ein Wasserstoffatom, einen Hydroxyrest oder einen Methylrest darstellen, oder K' ein Sauerstoffatom bedeutet, die punktierte Linie in Position 17 eine Epoxyfunktion darstellt und $R_a$ und $R_b$ die vorstehend angegebene Bedeutung besitzen.

**Patentansprüche für folgenden Vertragsstaat : ES**

**1.** Verfahren zur Herstellung von Hydrocortison der Formel (I)

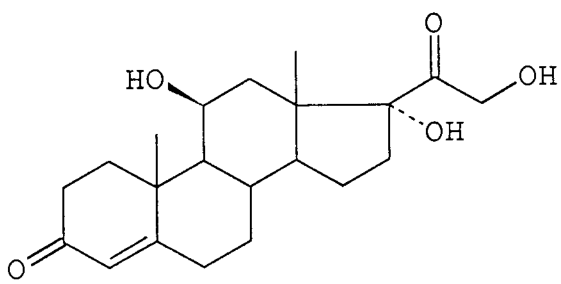

(I)

dadurch gekennzeichnet, daß man entweder ein Halohydrin der Formel (II)

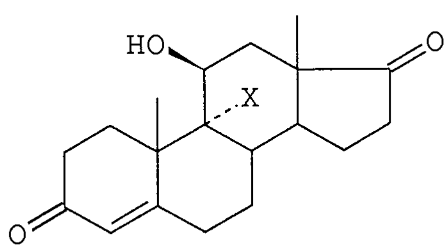

(II)

in der X ein Chlor-, Brom- oder Iodatom darstellt, einer Umlagerungsreaktion in Anwesenheit eines Alkohols oder eines Polyalkohols unterzieht, um nach Behandlung durch eine Säure das Produkt der Formel (III)

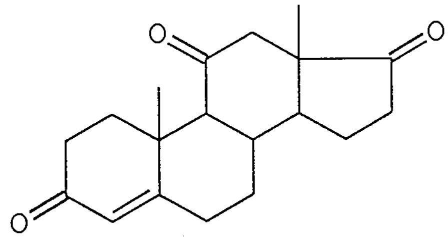

(III)

zu erhalten, bei dem man selektiv die Funktion 3-Oxo durch Einwirkung eines Thiols oder eines Dithiols der Formel

$HO-(CH_2)_n-SH$ oder $HS-(CH_2)_n-SH$

schützt, in der n gleich 2 oder 3 ist, um eine Verbindung der Formel (IV)

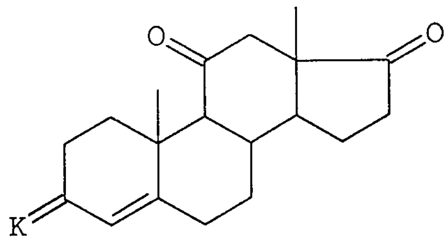

(IV)

zu erhalten, in der K eine Schutzgruppe der Formel

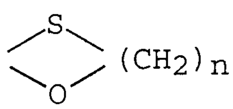

oder

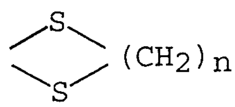

in der n wie vorstehend definiert ist, für den Rest 3-Oxo darstellt,
oder eine wie vorstehend definierte Verbindung der Formel (II) durch ein selektives Blockierungsmittel für die wie vorstehend definierte Funktion 3-Oxo behandelt, um eine Verbindung der Formel (V)

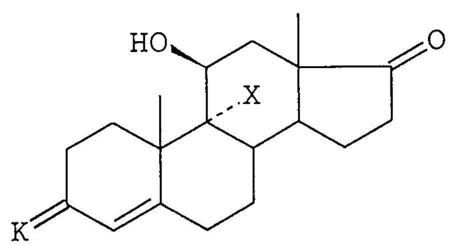

(V)

zu erhalten, in der X und K wie vorstehend definiert sind, die man einer Umlagerungsreaktion in Anwesenheit eines Alkohols oder eines Polyalkohols unterzieht, um nach Behandlung durch eine Säure eine wie vorstehend definierte Verbindung der Formel (IV) zu erhalten, wonach man die genannte Verbindung der Formel (IV) mit einem Trihaloacetat der Formel

$Hal_3C-CO_2R$

in der Hal ein Chlor- oder Bromatom darstellt und R einen Alkylrest mit 1 bis 6 Kohlenstoffatomen, einen Aralkylrest mit 7 bis 15 Kohlenstoffatomen oder einen Silylrest bedeutet, in Anwesenheit von Zink und einer Lewis-Säure behandelt, um eine Verbindung der Formel (VI)

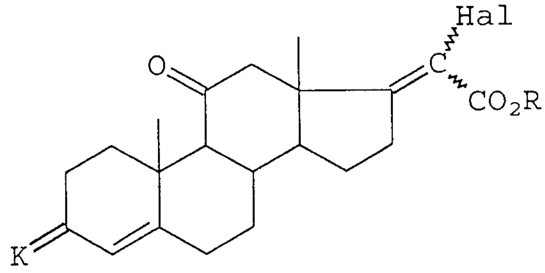

(VI)

zu erhalten, in der K, Hal und R wie vorstehend definiert sind, die man im basischen Medium mit einem Phenol der Formel

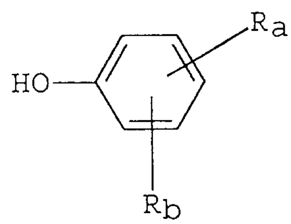

behandelt, in der $R_a$ und $R_b$, gleich oder verschieden, ein Wasserstoffatom, einen Hydroxyrest, einen Alkylrest mit 1 bis 4 Kohlenstoffatomen oder einen Alkoxyrest mit 1 bis 4 Kohlenstoffatomen darstellen, um eine Verbindung der Formel (VII)

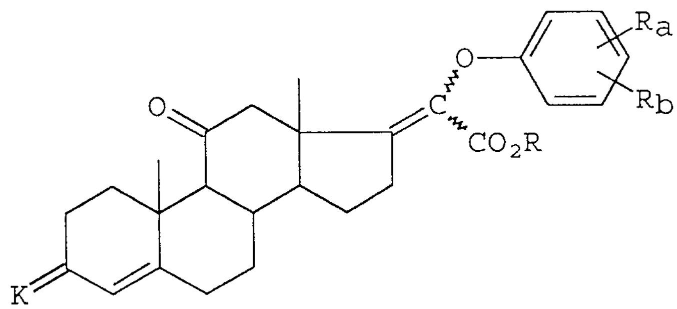

(VII)

zu erhalten, in der K, R, $R_a$ und $R_b$ wie vorstehend definiert sind, das man der Einwirkung eines Reduktionsmittels unterzieht, um eine Verbindung der Formel (VIII)

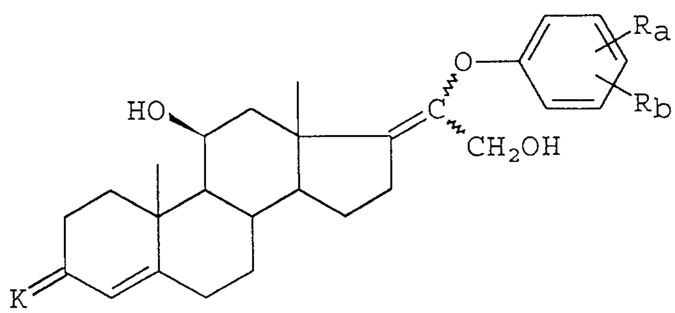

(VIII)

zu erhalten, in der K, $R_a$ und $R_b$ wie vorstehend definiert sind, bei der man von der Funktion 3-Oxo die Schutzgruppe entfernt, um eine Verbindung der Formel (IX)

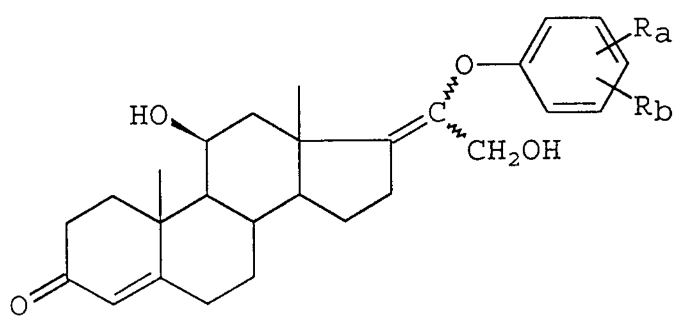

(IX)

zu erhalten, in der $R_a$ und $R_b$ wie vorstehend definiert sind, die man mit einem Epoxidierungsmittel behandelt, um eine Verbindung der Formel (X)

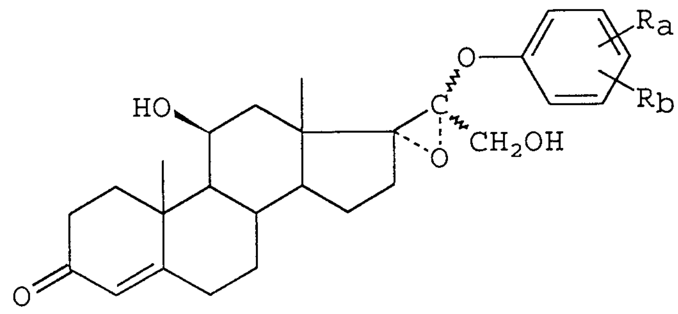

(X)

zu erhalten, in der $R_a$ und $R_b$ wie vorstehend definiert sind, die man dann im sauren Medium hydrolysiert, um die erwartete Verbindung der Formel (I) zu erhalten.

2. Verfahren zur Herstellung von Hydrocortison nach Anspruch 1, dadurch gekennzeichnet, daß man ein Halohydrin der Formel (II)

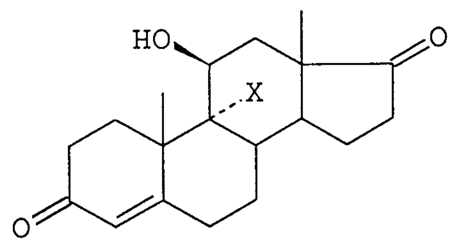

(II)

in der X wie in Anspruch 1 definiert ist, einer Umlagerungsreaktion in Anwesenheit eines Alkohols oder eines Polyalkohols unterzieht, um nach Behandlung durch eine Säure die Verbindung der Formel (III)

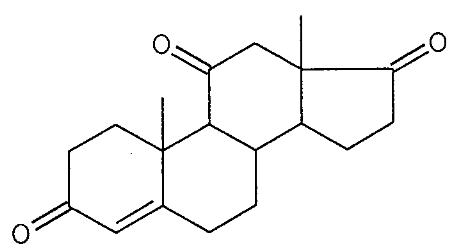

(III)

zu erhalten, bei der man selektiv die Funktion 3-Oxo durch Einwirkung eines Thiols oder eines Dithiols der Formel

$HO-(CH_2)_n-SH$ oder $HS-(CH_2)_n-SH$

schützt, in der n wie in Anspruch 1 definiert ist, um eine Verbindung der Formel (IV)

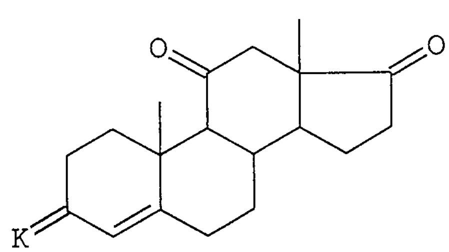

(IV)

zu erhalten, in der K wie in Anspruch 1 definiert ist, die man mit einem Reaktionspartner der Formel

$Hal_3C\text{-}CO_2R$

behandelt und anschließend die Synthese wie in Anspruch 1 beschrieben fortsetzt.

**3.** Verfahren zur Herstellung von Hydrocortison nach Anspruch 1, dadurch gekennzeichnet, daß man eine wie in Anspruch 1 definierte Verbindung der Formel (II) durch ein selektives Schutzmittel für die wie in Anspruch 1 definierte Funktion 3-Oxo behandelt, um eine Verbindung der Formel (V)

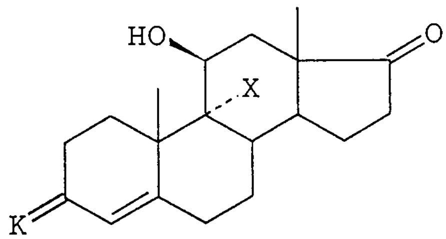

(V)

zu erhalten, in der X und K wie in Anspruch 1 definiert sind, die man einer Umlagerungsreaktion in Anwesenheit eines Alkohols oder eines Polyalkohols unterzieht, um nach Behandlung durch eine Säure eine wie in Anspruch 1 definierte Verbindung der Formel (IV) zu erhalten, wonach man die genannte Verbindung der Formel (IV) mit einem Reaktionspartner der Formel

$Hal_3C\text{-}CO_2R$

behandelt und anschließend die Synthese wie in Anspruch 1 beschrieben fortsetzt.

**4.** Verfahren nach Anspruch 1, 2 oder 3, dadurch gekennzeichnet, daß in der Verbindung der Formel (II) X ein Bromatom darstellt.

**5.** Verfahren nach Anspruch 1, dadurch gekennzeichnet, daß die Umlagerung des Halohydrins in Anwesenheit von Ethylenglycol, angewendet im Überschuß, durchgeführt wird.

**6.** Verfahren nach irgendeinem der Ansprüche 1 bis 5, dadurch gekennzeichnet, daß die Umlagerung in Anwesenheit eines Co-Lösungsmittels mit einem Siedepunkt von unter 100 °C, unter dessen Rückfluß durchgeführt wird.

**7.** Verfahren nach Anspruch 6, dadurch gekennzeichnet, daß das Co-Lösungsmittel Ethylacetat ist.

**8.** Verfahren nach irgendeinem der Ansprüche 1 bis 3, dadurch gekennzeichnet, daß man die selektive Blockierung der Funktion 3-Oxo mit Hilfe von Ethandithiol in Anwesenheit von konzentrierter Chlorwasserstoff- oder Bromwasserstoffsäure in katalytischer Menge oder von Bortrifluorid-etherat durchführt.

**9.** Verfahren nach Anspruch 1 oder 3, dadurch gekennzeichnet, daß man die Blockierung der Funktion 3-Oxo und die anschließende Umlagerung des Halohydrins ohne Isolierung des Zwischenproduktes der Formel (V) durchführt.

**10.** Verfahren nach irgendeinem der Ansprüche 1 bis 3, dadurch gekennzeichnet, daß das Trihaloacetat ein Trichloracetat von Methyl oder Ethyl ist.

**11.** Verfahren nach irgendeinem der Ansprüche 1 bis 3 und 10, dadurch gekennzeichnet, daß die Lewis-Säure Titantetrachlorid ist.

**12.** Verfahren nach irgendeinem der Ansprüche 1 bis 3, dadurch gekennzeichnet, daß in dem Phenol der Formel

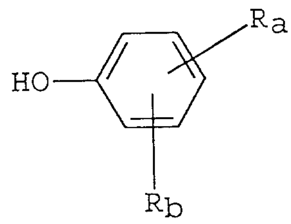

$R_a$ und $R_b$ ein Wasserstoffatom, einen Hydroxyrest oder einen Methylrest darstellen.

**13.** Verfahren nach irgendeinem der Ansprüche 1 bis 3, dadurch gekennzeichnet, daß das Reduktionsmittel ein Aluminiumhydrid oder ein Alkaliborhydrid ist.

**14.** Verfahren nach irgendeinem der Ansprüche 1 bis 3 und 8, dadurch gekennzeichnet, daß die Abspaltung der Schutzgruppe von der Funktion 3-Oxo entweder durch Einwirkung von Iod in Anwesenheit einer Base oder von Iod in katalytischer Menge in Anwesenheit eines Oxidationsmittels realisiert wird.

**15.** Verfahren nach irgendeinem der Ansprüche 1 bis 3, dadurch gekennzeichnet, daß das Epoxidierungsmittel eine Persäure und das Hydrolysemittel eine wäßrige Mineralsäure ist.